# EUROPÄISCHE PATENTSCHRIFT

(11) **EP 0 560 722 B1**
(45) Veröffentlichungstag und Bekanntmachung des Hinweises auf die Patenterteilung: **17.11.1999**
(21) Anmeldenummer: 93810160.7
(22) Anmeldetag: 04.03.1993
(51) Int. Cl.: C07D 498/04, C07D 487/04, B41M 5/145, B41M 5/30

(54) **Chromogene Lactamverbindungen, ihre Herstellung und Verwendung**
Chromogenic lactam compounds, their production and use
Composés lactames chromogènes, leur préparation et leur utilisation

(30) Priorität: 13.03.1992 CH 81592
(43) Veröffentlichungstag der Anmeldung: 15.09.1993
(73) Patentinhaber: Ciba Specialty Chemicals Holding Inc., 4057 Basel (CH)
(72) Erfinder: Baumann, Hans, Dr., CH-4104 Oberwil (CH); Phaff, Rox, Dr., CH-4452 Itingen (CH)

(56) Entgegenhaltungen:
- EP-A- 0 475 908
- US-A- 3 509 147
- JOURNAL OF THE CHEMICAL SOCIETY 1965, LETCHWORTH GB Seiten 2165 - 2173 E. ABRAMOWITZ ET AL 'Phthalanthranilic acid, its structure and reaction in Friedel-Crafts syntheses'

## Beschreibung

Die vorliegende Erfindung betrifft chromogene Lactamverbindungen, Verfahren zu ihrer Herstellung und ihre Verwendung in druck- oder wärmeempfindlichen Aufzeichnungsmaterialien.

Die erfindungsgemässen Lactamverbindungen entsprechen der allgemeinen Formel I 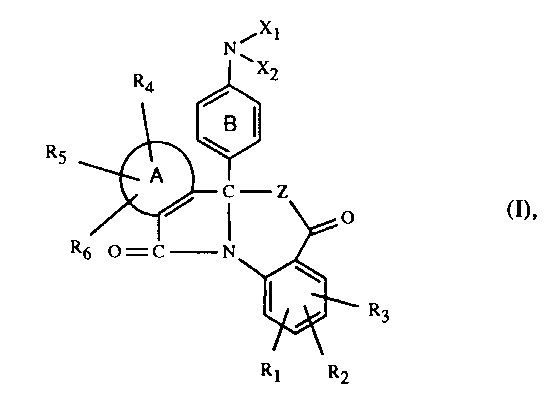 worin
- der Ring A: einen aromatischen oder heteroaromatischen Rest mit 6 Ringatomen, der einen annellierten Benzol-Ring aufweisen kann;
- der Ring B: einen gegebenenfalls durch Halogen, C₁-C₆-Alkyl, C₁-C₆-Alkoxy, Benzyloxy, C₁-C₆-Alkylcarbonylamino, Mono- oder Di-C₁-C₆-Alkylamino substituierten Benzolkern;
- Z: 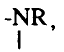 oder O;
- R: Wasserstoff; unsubstituiertes oder durch Halogen, Hydroxy, Cyano, Benzoyl, Formyl, C₁-C₆-Alkylcarbonyl, C₁-C₆-Alkylsulfonyl, C₁-C₆-Alkoxysulfonyl, Phenylsulfonyl, Phenoxysulfonyl, wobei Benzoyl und Phenylsulfonyl durch Halogen, Methyl, Methoxy oder Ethoxy substituiert sein können, Di-C₁-C₆-alkylamino oder C₁-C₆-Alkoxy substituiertes C₁-C₆-Alkyl; Cycloalkyl mit 5 bis 10 Kohlenstoffatomen; unsubstituiertes oder durch Halogen, Cyano, Nitro, C₁-C₄Halogenalkyl, C₁-C₆-Alkyl, C₁-C₆-Alkoxy, C₁-C₆-Alkoxycarbonyl oder Formyl, C₁-C₆-Alkylcarbonyl, Benzoyl, C₁-C₆-Alkylsulfonyl, C₁-C₆-Alkoxysulfonyl, Phenylsulfonyl, Phenoxysulfonyl, wobei Benzoyl und Phenylsulfonyl durch Halogen, Methyl, Methoxy oder Ethoxy substituiert sein können, ringsubstituiertes Naphthyl, Phenyl, Naphthyl-C₁-C₆-Alkyl oder Phenyl-C₁-C₄-alkyl; Benzoyl, Formyl, C₁-C₆-Alkylcarbonyl, C₁-C₆-Alkylsulfonyl, C₁-C₆-Alkoxysulfonyl, Phenylsulfonyl oder Phenoxysulfonyl, wobei Benzoyl und Phenylsulfonyl durch Halogen, Methyl, Methoxy oder Ethoxy substituiert sein können; N-C₁-C₆-Alkylcarbamoyl; oder gegebenenfalls ringsubstituiertes N-Naphthylcarbamoyl oder N-Phenylcarbamoyl;
- R₁, R₂ und R₃: unabhängig voneinander Wasserstoff; C₁-C₆-Alkyl; C₁-C₆-Alkoxy; C₁-C₆-Alkylthio;C₁-C₆-Alkylsulfonyl; Sulfo; Hydroxy; Nitro; Halogen; Amino; Mono-C₁-C₆-Alkylamino; Di-C₁-C₆-Alkylamino; Naphthylamino oder Phenylamino; Naphthyl-C₁-C₆-alkylamino; Phenyl-C₁-C₆-alkylamino; Dinaphthyloder Diphenylamino; (Naphthyl-C₁-C₆-alkyl)-alkylamino oder (Phenyl-C₁-C₆-alkyl)-alkylamino;(Naphthyl-C₁-C₆-alkyl)-C₁-C₆-alkylamino oder (Phenyl-C₁-C₆-alkyl)-C₁-C₆-alkylamino; Di-(naphthyl-C₁-C₆-alkyl)- oder Di-(phenyl-C₁-C₆-alkyl)-amino; oder einen über ein Ringstickstoffatom gebundenen Pyrrolidino-, Piperidino-, Pipecolino-, Morpholino-, Thiomorpholino-, Piperazino-, N-Methylpiperazino- oder N-Phenylpiperazinorest;
- R₂ und R₃: gemeinsam eine unsubstituierte oder durch C₁-C₆-Alkyl substituierte vicinal gebundene Methylendioxy- oder 1,2-Ethylendioxygruppe;
- R₄, R₅ und R₆: unabhängig voneinander Wasserstoff; Halogen; Cyano; Nitro; C₁-C₆-Alkyl; C₁-C₆-Alkylthio; C₁-C₆-Alkylcarbonyl; C₁-C₆-Alkoxycarbonyl; Amino; Mono-C₁-C₆-Alkylamino; Di-C₁-C₆-Alkylamino; Naphthyl- oder Phenylamino; Naphthyloder Phenyl-C₁-C₆-alkylamino; Dinaphthyl- oder Diphenylamino; Naphthyl- oder Phenyl-C₁-C₆-alkylamino; (Naphthyl-C₁-C₆-alkyl)- oder (Phenyl-C₁-C₆-alkyl)-C₁-C₆-alkylamino; Di-(naphthyl-C₁-C₆-alkyl)- oder Di-(phenyl-C₁-C₆-alkyl)-amino; C₅-C₁₀-Cycloalkylamino; DiC₅-C₁₀-cycloalkylamino; C₁-C₆-Alkyl C₅-C₁₀cycloalkylamino; C₁-C₆-Alkoxy; C₁-C₆-Alkylthio; C₁-C₆-Alkylsulfonyl; oder einen über ein Ringstickstoffatom gebundenen Pyrrolidino-, Piperidino-, Pipecolino-, Morpholino-, Thiomorpholino-, Piperazino-, N-Methylpiperazino- oder N-Phenylpiperazinorest;
- X₁ und X₂,: unabhängig voneinander, Wasserstoff; unsubstituiertes oder durch Halogen, Hydroxy, Cyano, Tetrahydrofuryl oder C₁-C₆-Alkoxy substituiertes Alkyl mit höchstens 12 Kohlenstoffatomen; Formyl, C₁-C₆-Alkylcarbonyl, Benzoyl, C₁-C₆-Alkylsulfonyl, C₁-C₆-Alkoxysulfonyl, Phenylsulfonyl, Phenoxysulfonyl, wobei Benzoyl und Phenylsulfonyl durch Halogen, Methyl, Methoxy oder Ethoxy substituiert sein können; Cycloalkyl mit 5 bis 10 Kohlenstoffatomen; oder unsubstituiertes oder durch Halogen, Cyano, Nitro, Trifluormethyl, C₁-C₆-Alkyl, C₁-C₆-Alkoxy, C₁-C₆-Alkoxycarbonyl, -NX'X" oder 4-X'X"N-phenylamino ringsubstituiertes Naphthyl-C₁-C₆-alkyl, Phenyl-C₁-C₆-alkyl, Naphthyl oder Phenyl, worin X' und X", unabhängig voneinander, Wasserstoff, C₁-C₆-Alkyl, Cyclohexyl, Benzyl oder Phenyl darstellen; oder
- X₁ und X₂: zusammen mit dem gemeinsamen Stickstoffatom einen Pyrrolidino-, Piperidino-, Pipecolino-, Morpholino-, Thiomorpholino-, Piperazino-, N-Methylpiperazino- oder N-Phenylpiperazinorest
bedeuten.

Als 6-gliedriger aromatischer Ring stellt A vorzugsweise einen Benzolring, als 6-gliedriger heterocyclischer Ring insbesondere einen stickstoffhaltigen Heterocyclus mit aromatischem Charakter, wie z.B. einen Pyridin- oder Pyrazinring dar. Der Ring A kann auch einen annellierten aromatischen Ring, vorzugsweise einen Benzolring enthalten und stellt somit z.B. einen Naphthalin-, Chinolin- oder Chinoxalinring dar.

Die durch A wiedergegebenen bevorzugten 6-gliedrigen aromatischen oder heterocyclischen Reste sind der 2,3-Pyridino-, 3,4-Pyridino-, 2,3-Pyrazino-, 2,3-Chinoxalino-, 1,2-Naphthalino-, 2,3-Naphthalino- oder 1,2-Benzorest.

Der Ring B ist vorteilhafterweise, neben dem obligatorischen Substituenten -NX₁X₂ unsubstituiert oder durch Halogen, Niederalkyl, Niederalkoxy, Acetylamino Mononiederalkylamino oder Diniederalkylamino mehrfach substituiert. Besonders bevorzugt ist der Ring B ein unsubstituierter oder durch Niederalkoxy substituierter Phenylrest.

Vorteilhafterweise bedeutet der Substituent R in Z als 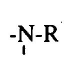 Wasserstoff, Niederalkyl, Cyano-Niederalkyl, Niederalkylcarbonyl wie Acetyl, Phenyl, Benzyl, N-Niederalkylcarbamoyl oder gegebenenfalls durch Halogen, Nitro, Trifluormethyl, Niederalkyl, Niederalkoxy oder Niederalkoxycarbonyl substituiertes N-Phenylcarbamoyl (Carbanilino). Vorzugsweise ist Z 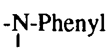 oder vor allem

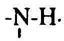

Stellen die Substituenten in Formel I Alkylgruppen dar, so können sie vorzugsweise geradkettig oder verzweigt sein mit einer Kettenlänge von bis zu 12 Kohlenstoffatomen. Beispiele solcher Alkylreste sind Methyl, Ethyl, n-Propyl, Isopropyl, n-Butyl, 1-Methylbutyl, sek.Butyl, tert.Butyl, Amyl, Isoamyl, n-Hexyl, 2-Ethylhexyl, n-Heptyl, n-Octyl, Isooctyl, 1,1,3,3-Tetramethylbutyl, n-Nonyl, Isononyl, 3-Ethylheptyl, Decyl oder n-Dodecyl.

Sind die Alkylreste in Formel I substituiert, so handelt es sich vor allem um Cyanoalkyl, Halogenalkyl, Hydroxyalkyl, Niederalkoxyalkyl jeweils vorzugsweise mit insgesamt 2 bis 8 Kohlenstoffatomen, wie z.B. 2-Cyanoethyl, 2-Chlorethyl, 2-Chlorpropyl, 2-Hydroxyethyl, 3-Hydroxypropyl, 2-Methoxyethyl, 2-Ethoxyethyl, 2,3-Dihydroxypropyl, 2-Hydroxy-2-chlorpropyl, 3-Methoxypropyl, 4-Methoxybutyl, Trichlormethyl, Trifluormethyl, Tetrafluoroethyl, Tetrachlorethyl oder 4-Propoxybutyl sowie bei X₁ und X₂ auch Tetrahydrofurfuryl.

Beispiele für Cycloalkyl sind Cyclopentyl, Cycloheptyl oder vorzugsweise Cyclohexyl. Die Cycloalkylreste können einen oder mehrere C₁-C₄Alkylreste, vorzugsweise Methylgruppen, enthalten und weisen insgesamt 5 bis 10 Kohlenstoffatome auf.

Als Aralkyl ist insbesondere Phenyl-C₁-C₄Alkyl, wie etwa Phenethyl, Phenylisopropyl oder vor allem Benzyl bevorzugt. Als Aryl stellen Substituenten der Formel I besonders Naphthyl oder in erster Linie Phenyl dar.

Bevorzugte Substituenten in der Aralkyl- und Arylgruppe der X-Reste sind z.B. Halogen, Cyano, C₁-C₄Alkyl, C₁-C₄Halogenalkyl, C₁-C₄Alkoxy, wie Methyl, Trifluormethyl, Methoxy oder Carbomethoxy. Bevorzugte Substituenten im Arylrest R sind z.B. Halogen, Methyl oder Methoxy. Beispiele für derartige araliphatische bzw. aromatische Reste sind Methylbenzyl, 2,4- oder 2,5-Dimethylbenzyl, Chlorbenzyl, Dichlorbenzyl, Cyanobenzyl, Tolyl, Xylyl, Chlorphenyl, Methoxyphenyl, Trifluormethylphenyl, 2,6-Dimethylphenyl oder Carbomethoxyphenyl.

Wenn X₁ und X₂ zusammen mit dem gemeinsamen Stickstoffatom einen heterocyclischen Rest darstellen, oder wenn einer der Substituenten R₁ bis R₆ ein über ein Stickstoffatom gebundenen ganz oder teilweise gesättigten Heterocyclus bedeutet, so ist dieser Pyrrolidino, Piperidino, Pipecolino, Morpholino, Thiomorpholino oder Piperazino, N-Methylpiperazino oder N-Phenylpiperazino. Bevorzugte gesättigte heterocyclische Reste sind Pyrrolidino, Piperidino oder Morpholino.

Die Substituenten X₁ und X₂ sind vorzugsweise Cyclohexyl, Tolyl, Benzyl, Phenyl, Cyano-Niederalkyl, z.B. 2-Cyanoethyl oder in erster Linie Niederalkyl, wie z.B. Methyl, Ethyl, n-Propyl, Isopropyl, n-Butyl oder Isoamyl. -NX₁X₂ ist bevorzugt auch Pyrrolidino, N-Niederalkyl-N-tetrahydrofurfurylamino, 4-Diniederalkylaminophenylamino oder 4-(4'-Phenylamino-phenylamino)-phenylamino.

Niederalkyl, Niederalkoxy und Niederalkylthio stellen vorzugsweise solche Gruppen oder Gruppenbestandteile dar, die 1 bis 6, insbesondere 1 bis 4 Kohlenstoffatome aufweisen. Beispiele für derartige Gruppen sind Methyl, Ethyl, n-Propyl, Isopropyl, n-Butyl, sek.Butyl, Amyl, Isoamyl oder Hexyl bzw. Methoxy, Ethoxy, Isopropoxy, Isobutoxy, tert.Butoxy oder Amyloxy bzw. Methylthio, Ethylthio, Propylthio oder Butylthio.

Halogen bedeutet beispielsweise Fluor, Brom, Iod oder, vorzugsweise, Chlor.

"Acyl" ist Formyl, Niederalkylcarbonyl, wie z.B. Acetyl oder Propionyl, oder Benzoyl. Weitere Acylreste können Niederalkylsulfonyl, wie z.B. Methylsulfonyl oder Ethylsulfonyl, Niederalkoxysulfonyl, wie z.B. Methoxysulfonyl oder Ethoxysulfonyl, sowie Phenylsulfonyl oder Phenoxysulfonyl sein. Benzoyl und Phenylsulfonyl können durch Halogen, Methyl, Methoxy oder Ethoxy substituiert sein.

Bevorzugt sind Verbindungen der Formel I, 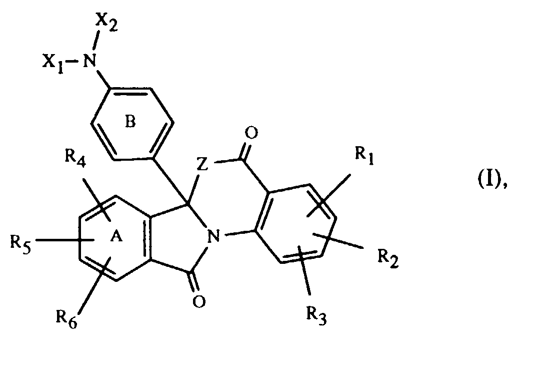 worin
- der Ring B: einen gegebenenfalls durch Halogen, C₁-C₆-Alkyl, C₁-C₆-Alkoxy, oder Benzyloxy substituierten Benzolkern;
- Z: 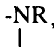 oder O;
- R: Wasserstoff; unsubstituiertes oder durch Halogen oder C₁-C₆-Alkoxy substituiertes C₁-C₆-Alkyl; Cycloalkyl mit 5 bis 10 Kohlenstoffatomen; unsubstituiertes oder durch Halogen, Cyano, Nitro, C₁-C₄Halogenalkyl, C₁-C₆-Alkyl oder C₁-C₆-Alkoxy ringsubstituiertes Phenyl oder Benzyl; oder Benzoyl, Formyl, C₁-C₆-Alkylcarbonyl, C₁-C₆-Alkylsulfonyl, C₁-C₆-Alkoxysulfonyl, Phenylsulfonyl oder Phenoxysulfonyl, wobei Benzoyl und Phenylsulfonyl durch Halogen, Methyl, Methoxy oder Ethoxy substituiert sein können;
- R₁, R₂ und R₃: unabhängig voneinander Wasserstoff; C₁-C₆-Alkyl; C₁-C₆-Alkoxy; C₁-C₆-Alkylthio;C₁-C₆-Alkylsulfonyl; Sulfo; Hydroxy; Nitro; Halogen; Amino; Mono-C₁-C₆-Alkylamino; Di-C₁-C₆-Alkylamino; Naphthylamino oder Phenylamino; Naphthyl-C₁-C₆-alkylamino; Phenyl-C₁-C₆-alkylamino; Dinaphthyloder Diphenylamino; (Naphthyl-C₁-C₆-alkyl)-alkylamino oder (Phenyl-C₁-C₆-alkyl)-alkylamino;(Naphthyl-C₁-C₆-alkyl)-C₁-C₆-alkylamino oder (Phenyl-C₁-C₆-alkyl)-C₁-C₆-alkylamino; Di-(naphthyl-C₁-C₆-alkyl)-amino oder Diphenyl-C₁-C₆-alkylamino; oder einen über ein Ringstickstoffatom gebundenen Pyrrolidino-, Piperidino-, Pipecolino-, Morpholino-, Thiomorpholino-, Piperazino-, N-Methylpiperazino- oder N-Phenylpiperazinorest;
- R₂ und R₃: gemeinsam eine unsubstituierte oder durch C₁-C₆-Alkyl substituierte vicinal gebundene Methylendioxy- oder 1,2-Ethylendioxygruppe;
- R₄, R₅ und R₆: unabhängig voneinander Wasserstoff; Halogen; Cyano; Nitro; C₁-C₆-Alkyl; C₁-C₆-Alkylthio; C₁-C₆-Alkylcarbonyl; C₁-C₆-Alkoxycarbonyl; Amino; Mono-C₁-C₆-Alkylamino; Di-C₁-C₆-Alkylamino; Naphthyl- oder Phenylamino; Naphthyl-oder Phenyl-C₁-C₆-alkylamino; Dinaphthyl- oder Diphenylamino; (Naphthyl-C₁-C₆-alkyl)-amino oder (Phenyl-C₁-C₆-alkyl)amino; (Naphthyl-C₁-C₆-alkyl)- oder (Phenyl-C₁-C₆-alkyl)-C₁-C₆-alkylamino; Di-(naphthyl-C₁-C₆-alkyl)- oder Di-(phenyl-C₁-C₆-alkyl)-amino; C₅-C₁₀-Cycloalkylamino; DiC₅-C₁₀-cycloalkylamino; C₁-C₆-Alkyl-C₅-C₁₀ -cycloalkylamino; C₁-C₆-Alkoxy; C₁-C₆-Alkylthio; C₁-C₆-Alkylsulfonyl; oder einen über ein Ringstickstoffatom gebundenen Pyrrolidino-, Piperidino-, Pipecolino-, Morpholino-, Thiomorpholino-, Piperazino-, N-Methylpiperazino- oder N-Phenylpiperazinorest;
- X₁ und X₂,: unabhängig voneinander, Wasserstoff; unsubstituiertes oder durch Halogen, Hydroxy, Cyano, Tetrahydrofuryl oder C₁-C₆-Alkoxy substituiertes Alkyl mit höchstens 12 Kohlenstoffatomen; Formyl, C₁-C₆-Alkylcarbonyl, Benzoyl, C₁-C₆-Alkylsulfonyl, C₁-C₆-Alkoxysulfonyl, Phenylsulfonyl, Phenoxysulfonyl, wobei Benzoyl und Phenylsulfonyl durch Halogen, Methyl, Methoxy oder Ethoxy substituiert sein können; Cycloalkyl mit 5 bis 10 Kohlenstoffatomen; oder unsubstituiertes oder durch Halogen, Cyano, Nitro, Trifluormethyl oder C₁-C₆-Alkyl, ringsubstituiertes Naphthyl-C₁-C₆-alkyl, Phenyl-C₁-C₆-alkyl, Naphthyl oder Phenyl; oder
- X₁ und X₂: zusammen mit dem gemeinsamen Stickstoffatom einen Pyrrolidino-, Piperidino-, Pipecolino-, Morpholino-, Thiomorpholino-, Piperazino-, N-Methylpiperazino- oder N-Phenylpiperazinorest
bedeuten.

Besonders bevorzugt sind Verbindungen der Formel I, 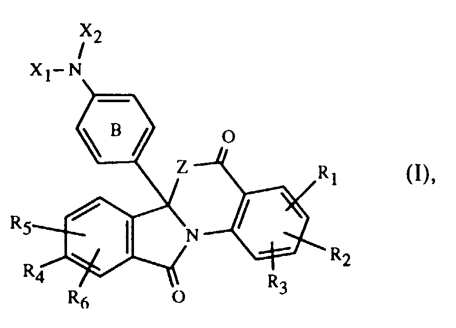 worin
- der Ring B: einen unsubstituierten oder gegebenenfalls durch Halogen oder C₁-C₄Alkyl, substituierten Benzolkern;
- Z: 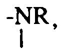 oder O;
- R: Wasserstoff;
- R₁: Wasserstoff; C₁-C₄Alkyl; C₁-C₄Alkoxy; Sulfo; Nitro; Halogen; Mono-C₁-C₄Alkyl-amino; Di-C₁-C₄Alkylamino; Phenylamino; Phenyl-C₁-C₄Alkylamino; Diphenylamino; Benzylamino; Benzyl-C₁-C₄Alkylamino; Dibenzylamino;
- R₂ und R₃: unabhängig voneinander Wasserstoff; C₁-C₄Alkyl; oder Halogen;
- R₂ und R₃: gemeinsam eine vicinal gebundene Methylendioxy- oder 1,2-Ethylendioxygruppe;
- R₄: Wasserstoff; Amino; Mono-C₁-C₄Alkylamino; Di-C₁-C₄Alkylamino; Phenylamino; Phenyl-C₁-C₄Alkylamino; Diphenylamino; Benzylamino; Benzyl-C₁-C₄Alkylamino; Dibenzylamino; Cycloalkylamino; Dicycloalkylamino; oder C₁-C₄Alkyl-cycloalkylamino;
- R₅ und R₆: unabhängig voneinander Wasserstoff; Halogen; Cyano; Nitro; C₁-C₄Alkyl; C₁-C₄Alkylthio; oder C₁-C₄Alkoxy;
- X₁ und X₂,: unabhängig voneinander, Wasserstoff; unsubstituiertes oder durch C₁-C₄Alkoxy substituiertes Alkyl mit höchstens 12 Kohlenstoffatomen; Cyclohexyl; der unsubstituiertes oder durch Halogen, C₁-C₄Alkyl, C₁-C₄Alkoxy, ringsubstituiertes Benzyl oder Phenyl; oder
- X₁ und X₂: zusammen mit dem gemeinsamen Stickstoffatom einen Pyrrolidino-, Piperidino-, Pipecolino-, Morpholino-, Thiomorpholino-, Piperazino-, N-Methylpiperazino- oder N-Phenylpiperazinorest
bedeuten.

Von den Verbindungen der Formel I sowie den als bevorzugt und besonders bevorzugt herausgestellten Verbindungen der Formel I sind die nachfolgenden Gruppen a) bis g) von besonderem Interesse:
a) Verbindungen der Formel I, worin
   - Z: O;
b) Verbindungen der Formel I, worin
   - R₁: wie zuvor definiert ist,
   - R₂: Wasserstoff, Halogen oder C₁-C₄Alkyl und
   - R₃: Halogen oder Wasserstoff;
c) Verbindungen der Formel I, worin
   - R₁: Mono-C₁-C₄Alkylamino; Di-C₁-C₄Alkylamino;
   - R₂: Wasserstoff oder C₁-C₄Alkyl und
   - R₃: Wasserstoff;
d) Verbindungen der Formel I, worin
   - R₄: C₁-C₄Alkoxy; Mono-C₁-C₄Alkylamino; Di-C₁-C₄Alkylamino; Phenylamino; Phenyl-C₁-C₄Alkylamino; Diphenylamino; Benzylamino; Benzyl-C₁-C₄Alkylamino; Dibenzylamino, C₅-C₁₀-Cycloalkylamino; DiC₅-C₁₀-cycloalkylamino; oder C₁-C₄Alkyl-C₅-C₁₀-cycloalkylamino;
e) Verbindungen der Formel I, worin
   - R₄: Mono-C₁-C₄Alkylamino, Di-C₁-C₄Alkylamino, Phenylamino, Phenyl-C₁-C₄Alkylamino, Diphenylamino, Benzylamino, Benzyl-C₁-C₄Alkylamino, Dibenzylamino;
f) Verbindungen der Formel I, worin
   - X₁ und X₂,: unabhängig voneinander, Wasserstoff, unsubstituiertes Alkyl mit höchstens 6 Kohlenstoffatomen, Benzyl oder Phenyl;
g) Verbindungen der Formel I, worin
   - X₁ und X₂,: unabhängig voneinander, unsubstituiertes Alkyl mit höchstens 6 Kohlenstoffatomen, Benzyl oder Phenyl;
   bedeutet.

Die erfindungsgemässen Lactamverbindungen der Formel (I) sind neue Verbindungen. Sie können nach an sich bekannten Methoden hergestellt werden.

Vorteilhafterweise werden die erfindungsgemässen Lactamverbindungen dadurch hergestellt, dass man eine Ketosäure der Formel 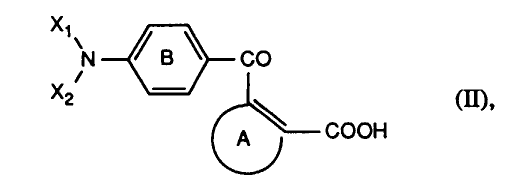 worin X₁, X₂, A und B wie zuvor definiert sind,
mit einer bifunktionellen Aminoverbindung der Formel 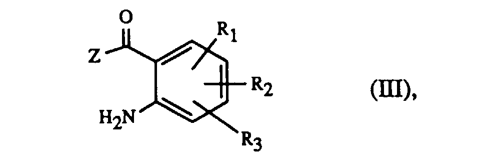 worin R₁, R₂, R₃ und Z wie zuvor definiert sind,
umsetzt.

Die Reaktion wird vorzugsweise in einem reaktionsinerten organischen Lösungsmittel bei erhöhter Temperatur, vorzugsweise bis zur Siedetemperatur, durchgeführt.

Geeignete organische Lösungsmittel, die das Reaktionsmedium bilden, sind cycloaliphatische oder vorzugsweise aromatische Kohlenwasserstoffe, wie z.B. Cyclohexan, Benzol, Toluol oder Xylol; Chlorkohlenwasserstoffe, wie z.B. Ethylenchlorid, Tetrachlorethylen oder Chlorbenzole, wie z.B. Chlorbenzol, Chlortoluol oder Dichlorbenzol; cyclische Ether, wie z.B. Dioxan oder Tetrahydrofuran; Dimethylsulfoxid oder Nitrile aliphatischer Monocarbonsäuren, wie z.B. Acetonitril, Propionitril oder Butyronitril. Auch Mischungen der genannten Lösungsmittel können verwendet werden. Bevorzugte Lösungsmittel sind Chlorbenzol, Chlortoluol und besonders Toluol.

Die Isolierung des Endproduktes erfolgt in allgemein bekannter Weise durch Trennung der gebildeten Wasserphase und Entfernung des Lösungsmittels oder durch Behandeln mit geeigneten organischen Lösungsmitteln, wie z.B. Methanol, Isopropanol oder Petrolether.

Die bifunktionellen Verbindungen der Formel (III) können je nach der Bedeutung von Z als Anthranilsäure, Anthranilsäureamide, Aminonaphthoesäure oder Aminonaphthoesäureamide eingesetzt werden.

Geeignete Aminoverbindungen der Formel (III) zur Umsetzung mit den Ketosäuren der Formel (II) sind z.B. Aminonaphthoesäure, 4-Nitroanthranilsäure, 3,5-Diiodoanthranilsäure, 3-Hydroxyanthranilsäure, 3-Methylanthranilsäure, 4-Chloranthranilsäure, 6-Methylanthranilsäure, 5-Bromanthranilsäure, 5-Chloranthranilsäure, 5-Iodanthranilsäure, 5-Hydroxyanthranilsäure, 5-Methylanthranilsäure, 4,5-Dimethylanthranilsäure, 5-Methoxyanthranilsäure, 5-Nitroanthranilsäure, 3,5-Dichloroanthranilsäure, 3,5-Dimethylanthranilsäure, 3,4-Methylendioxyanthranilsäure, 3-Nitroanthranilsäure, 3-Sulfoanthranilsäure, 3,4,5-Trimethoxyanthranilsäure oder 5-Fluoranthranilsäure.

Verbindungen der Formel (I), worin Z 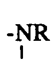 und R Acyl, N-Niederalkylcarbamoyl oder gegebenenfalls substituiertes N-Phenylcarbamoyl bedeuten, können auch dadurch hergestellt werden, dass man eine Lactamverbindung der Formel (I), worin 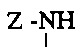 bedeutet, auf übliche Weise mit einem reaktiven funktionellen Derivat einer Carbonsäure oder einer Sulfonsäure, insbesondere Halogeniden oder Anhydriden z.B. Acetanhydrid, Acetylchlorid, Acetylbromid, Benzoylchlorid, Benzolsulfonylchlorid oder auch mit Isocyanaten, wie z.B. Niederalkylisocyanaten, Phenylisocyanat, Halogenphenylisocyanat oder Niederalkylphenylisocyanat umsetzt.

Die Lactamverbindungen der Formel (I) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie je nach der Bedeutung von Z und dem verwendeten Entwickler intensive gelbe, rote, violette, grün-blaue, blaue oder grüne Farbtöne, die sublimations- und lichtecht sind. Die Lactame der Formel (I) sind auch sehr wertvoll im Gemisch mit einem oder mehreren anderen bekannten Farbbildnern, z.B. 3,3-(Bis-aminophenyl-)-phthaliden, wie CVL, 3-Indolyl-3-aminophenyl-aza- oder -diazaphthaliden, (3,3-Bis-indolyl-)-phthaliden, 3-Aminofluoranen, 2,6-Diaminofluoranen, 2,6-Diamino-3-methyl-fluoranen, 3,6-Bisalkoxy-fluoranen, 3,6-Bisdiarylaminofluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um marineblaue, graue oder schwarze Färbungen zu ergeben.

Die Lactamverbindungen der Formel (I) zeigen sowohl auf aktivierten Tonen als auch auf phenolischen Substraten eine ausgezeichnete Farbintensität und Lichtechtheit. Sie eignen sich vor allem als Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie pH stabil und in den Kapselölen hervorragend löslich sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, die mindestens einen Farbbildner der Formel (I) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxid, Aluminiumoxid, Aluminiumsulfat, Aluminiumphosphat, Zinkchlorid, Zinknitrat, Zirkondioxid, aktiviertes Kaolin oder irgendein beliebiger Ton. Als Entwickler können auch sauer reagierende, organische Verbindungen, wie z.B. gegebenenfalls ringsubstituierte Phenole, Resorcine, Salicylsäuren, wie z.B. 3,5-Bis-(α,α-dimethylbenzyl-)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate, wie z.B. Zink-3,5-bis-α-methylbenzylsalicylat oder die Kondensationsprodukte von p-substituierten Phenolen mit Formaldehyd. Die letzteren können auch mit Zink modifiziert sein. Die Zinksalicylate sind z.B. in EP-A-181 283 oder DE-A-2 242 250 beschrieben.

Die Entwickler können zusätzlich auch im Gemisch mit an sich unreaktiven oder wenig reaktiven Pigmenten oder weiteren Hilfsstoffen wie Kieselgel oder UV-Absorbern, wie z.B. 2-(2'-Hydroxyphenyl-)benztriazolen oder 2-Hydroxyphenyl-1,2,3-triazinen eingesetzt werden. Beispiele für solche Pigmente sind: Talk, Titandioxid, Aluminiumoxid, Aluminiumhydroxid, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmäßig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Paraffin, Benzol oder Diphenyl, wie Chlorparaffin, Trichlorbenzol, Monochlordiphenyl, Dichlordiphenyl oder Trichlordiphenyl, Ester, wie z.B. Tricresylphosphat, Di-n-butylphthalat, Dioctylphthalat, Trichlorethylphosphat, aromatische Ether, wie Benzylphenylether, Kohlenwasserstofföle, wie Paraffin oder Kerosin, aromatische Kohlenwasserstoffe, z.B. mit Isopropyl, Isobutyl, sek.Butyl oder tert.Butyl alkylierte Derivate von Diphenyl, Naphthalin oder Terphenyl, Dibenzyltoluol, partiell hydriertes Terphenyl, mono- bis tetra-C₁-C₃alkylierte Diphenylalkane, Dodecylbenzol, benzylierte Xylole, Phenylxylylethan oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Lactame dadurch aus, dass sie gut löslich sind und pH-Beständigkeit z.B. in einem pH-Bereich von 4 bis 10 zeigen.

Die Kapselwände können durch Koazervationskräfte gleichmäßig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052, 4,100,103 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid, Polyharnstoff oder Polyurethan.

Die Farbbildner der Formel (I) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Übertragungsblattes und der Elektronenakzeptor (Farbentwickler) in Form einer Schicht auf der Vorderseite eines Empfangsblattes vorhanden sind. Eine andere Anordnung der Bestandteile besteht darin, dass die den Farbbildner enthaltenden Mikrokapseln und der Entwickler in oder auf dem gleichen Blatt in Form einer oder mehrerer Einzelschichten vorliegen oder der Entwickler im Trägermaterial eingebaut ist.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acryl-homo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet. Schichtträger kann auch eine Kunststoffolie sein.

Vorzugsweise besteht das Durchschreibematerial auch darin, dass es eine kapselfreie, den Farbbildner enthaltende Schicht und eine farbentwickelnde Schicht, die als Farbentwickler mindestens ein anorganisches Metallsalz eines mehrwertigen Metalls, vor allem Halogenide oder Nitrate, wie z.B. Zinkchlorid, Zinnchlorid, Zinknitrat oder deren Gemische enthält, aufweist.

Die Verbindungen der Formel (I) können auch als Farbbildner in einem thermoreaktiven Aufzeichnungsmaterial verwendet werden. Dieses enthält in der Regel mindestens einen Schichtträger, einen oder mehrere Farbbildner, und einen Elektronenakzeptor und gegebenenfalls auch ein Bindemittel und/oder Wachs. Gewünschtenfalls können auch Aktivatoren oder Sensibilisatoren, z.B. Benzyldiphenyl, im Aufzeichnungsmaterial vorhanden sein.

Thermoreaktive Aufzeichnungssysteme umfassen z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Die Schicht bzw. Schichten werden in spezifischen Bezirken mittels Wärme erweicht, worauf sich sofort in den erwärmten Teilen die erwünschte Farbe entwickelt.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 1 251 348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenylsulfon, 4'-Hydroxy-4-methyldiphenylsulfon, 4'-Hydroxy-4-isopropoxydiphenylsulfon, 4,4'-Cyclohexylidendiphenol, 4,4'-Isopropylidendiphenol, 4,4'-Isopropyliden-bis-(2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Hydroxyphthalsäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Lactame und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Um die Stabilität des wärmeempfindlichen Aufzeichnungsmaterials oder die Bilddichte des entwickelten Bildes zu gewährleisten, kann das Material mit einer zusätzlichen Schutzschicht versehen sein. Derartige Schutzschichten bestehen in der Regel aus wasserlöslichen und/oder wasserunlöslichen Harzen, die herkömmliche Polymermaterialien oder wässrige Emulsionen von diesen Polymermaterialien sind.

Die thermoreaktiven Schichten und Harzschichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxid, Calciumcarbonat (z.B. Kreide), Tone oder auch organische Pigmente, wie z.B. Harnstoff-Formaldehydpolymerisate enthalten. Um zu bewirken, dass nur innerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Stearinsäureamid, Bis-stearoylethylendiamid, Phthalsäureanhydrid, Metallstearate, wie z.B. Zinkstearat, Phthalsäurenitril, Dimethylterephthalat, Dibenzylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formel (I) ist die Herstellung eines Farbbildes mittels photohärtbarer Mikrokapseln, wie sie z.B. in der DE-OS 3 247 488 beschrieben sind.

Die folgenden Beispiele erläutern die Erfindung. Die angegebenen Prozentsätze beziehen sich, wenn nichts anderes angegeben ist, auf das Gewicht.

In den Beispielen werden die einzelnen Substituenten wie folgt numeriert: Wenn A für einen Benzolring steht, betrifft die Erfindung je nach der Bedeutung von Z Isoindolo-[1,2-a]-3,1-benzoxazine (Z = O) oder Isoindolo[1,2-a]-3,1-chinazoline (Z = NR).

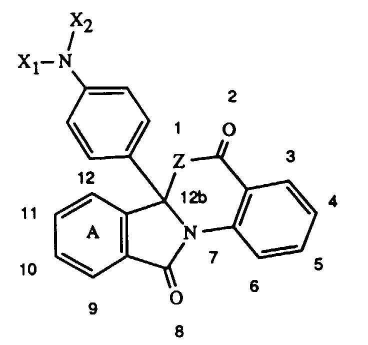

Analog dazu werden die Verbindungen der Formel (I) benannt, in denen der Ring A ein heteroaromatischer Rest oder ein anellierter Benzolrest oder ein anellierter heteroaromatischer Rest ist.

### Beispiel 1: 10-Dimethylamino-12b-(4'-Dimethylaminophenyl)-2,7,8,12b-tetrahydro-iso-indolo[1,2-a]-3,1-benzoxazin-2,8-dion

18,6 Teile 4,4'-Bis-dimethylaminobenzophenon-2-carbonsäure,
9,9 Teile Anthranilsäure und
0,5 Teile p-Toluolsulfonsäure werden in
150 ml Chlorbenzol

3,5 h am Wasserabscheider zum Sieden erhitzt. Danach wird mit Triethylamin neutralisiert und die Lösung über Alox filtriert. Die resultierende gelbe Lösung wird eingeengt, der Rückstand in wenig heissem Chlorbenzol gelöst. Anschliessend lässt man abkühlen. Das auskristallisierte Produkt wird abfiltriert und getrocknet.

Man isoliert 19,6 Teile der Verbindung der Formel 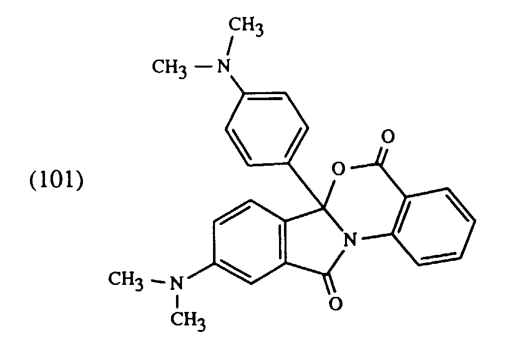 als Kristalle
Schmelzpunkt: 227 - 8°C

Eine toluolische Lösung dieser Verbindung ergibt in Kontakt mit saurem Ton eine grün-blaue Farbe.

### Beispiel 2: 10-Dimethylamino-12b-(4'-Diethylaminophenyl)-2,7,8,12b-tetrahydroiso-indolo[1,2a]-3,1-benzoxazin-2,8-dion

19,5 Teile 4-Diethylamino-4'-dimethylamino-2'-carbonsäure,
9,9 Teile Anthranilsäure und
0,5 Teile p-Toluolsulfonsäure werden in
150 ml Chlorbenzol

während 4 Stunden am Wasserabscheider zum Sieden erhitzt. Danach wird mit Triethylamin neutralisiert und die Lösung über Alox filtriert. Die resultierende Lösung wird eingedampft und der Rückstand aus wenig heissem Toluol umkristallisiert. Man erhält 17,2 Teile der Verbindung der Formel 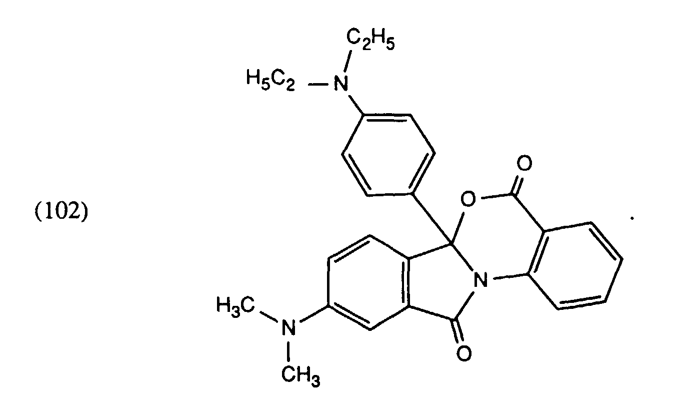 Schmelzpunkt: 211-213°C

Analog zu dem vorstehenden Beispicl können die Verbindungen der Tabelle 1 hergestellt werden:

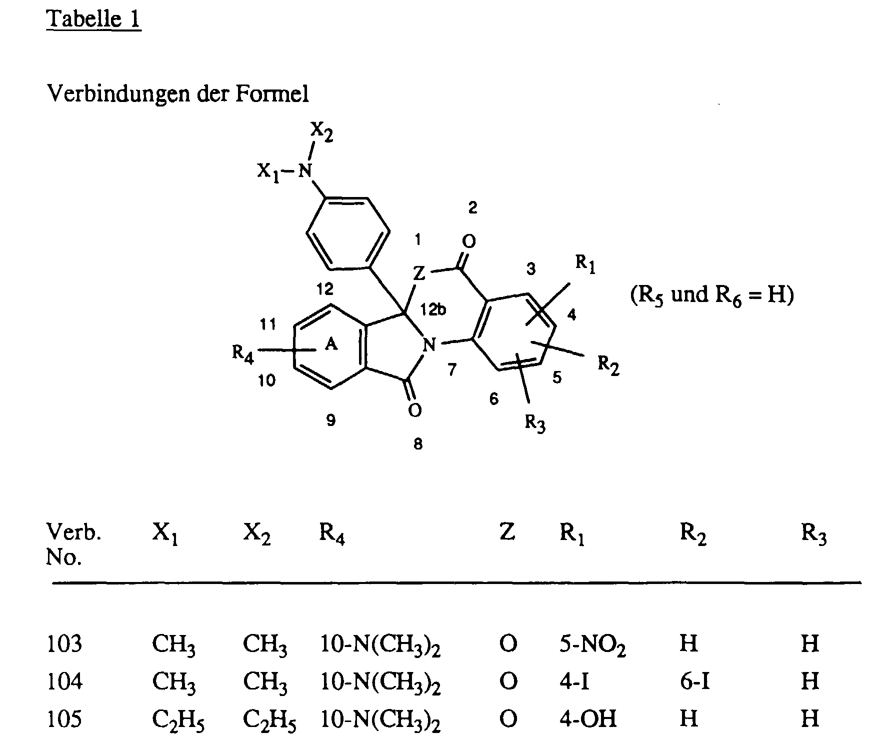

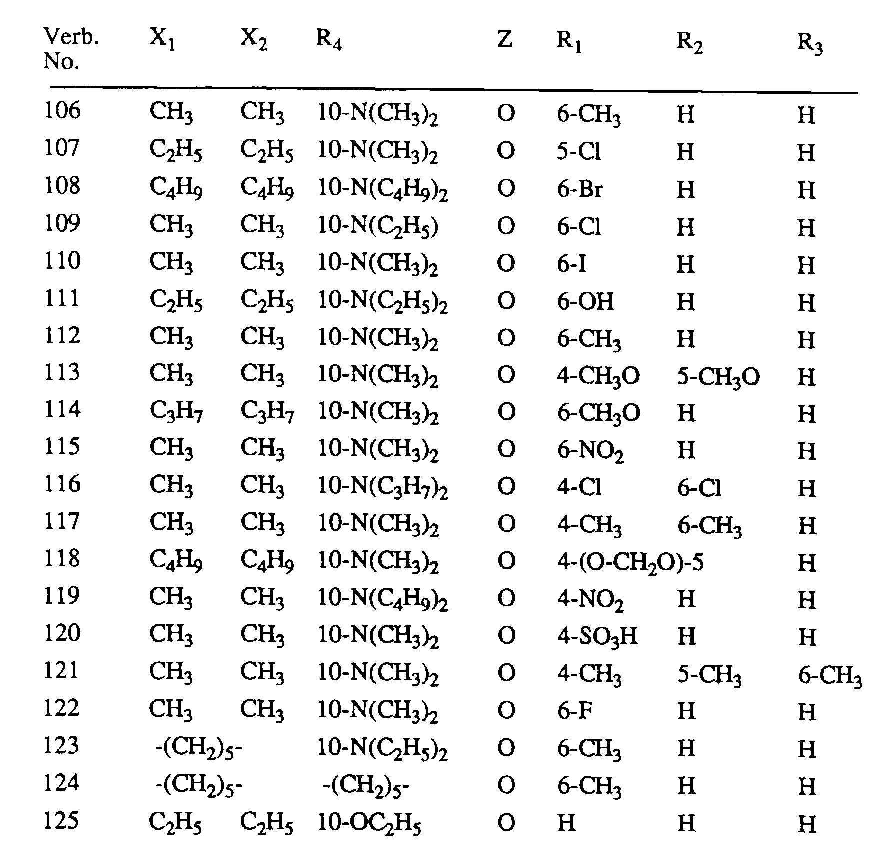

Von den in der Tabelle aufgeführten Verbindungen lassen sich die λₘₐₓ-Werte bestimmen, wenn sie auf saurem Ton appliziert werden. Die Werte liegen zwischen 500 und 550 nm (gemessen in Remission).

### Anwendungsbeispiele

### Anwendungsbeispiel 1: Herstellung eines druckempfindlichen Kopierpapiers.

Eine Lösung von 3 g der Lactamverbindung (101) aus Beispiel 1 in 80 g Diisopropylnaphthalin und 17 g Kerosin wird auf an sich bekannte Weise mit Gelatine und Carboxymethylcellulose durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich auf dem mit dem Entwickler beschichteten Blatt eine intensive blaue Kopie, die ausgezeichnet lichtecht ist.

### Anwendungsbeispiel 2: Herstellung eines druckempfindlichen Kopierpapiers

1 g der Lactamverbindung (101) gemäss Beispiel 1 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich auf dem mit dem Farbbildner beschichteten Blatt eine intensive und lichtechte blaue Farbe.

## Patentansprüche

1. Lactamverbindung der allgemeinen Formel I 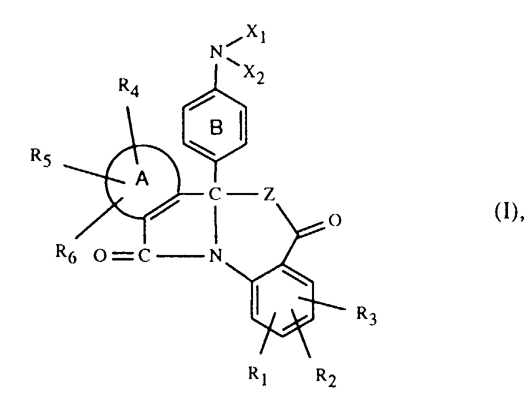 worin
der Ring A einen aromatischen oder heteroaromatischen Rest mit 6 Ringatomen, der einen annellierten Benzol-Ring aufweisen kann;
der Ring B einen gegebenenfalls durch Halogen, C₁-C₆-Alkyl, C₁-C₆-Alkoxy, Benzyloxy, C₁-C₆-Alkylcarbonylamino, Mono- oder Di-C₁-C₆-Alkylamino substituierten Benzolkern;
Z 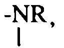 oder O;
R Wasserstoff; unsubstituiertes oder durch Halogen, Hydroxy, Cyano, Benzoyl, Formyl, C₁-C₆-Alkylcarbonyl, C₁-C₆-Alkylsulfonyl, C₁-C₆-Alkoxysulfonyl, Phenylsulfonyl, Phenoxysulfonyl, wobei Benzoyl und Phenylsulfonyl durch Halogen, Methyl, Methoxy oder Ethoxy substituiert sein können, Di-C₁-C₆-alkylamino oder C₁-C₆-Alkoxy substituiertes C₁-C₆-Alkyl; Cycloalkyl mit 5 bis 10 Kohlenstoffatomen; unsubstituiertes oder durch Halogen, Cyano, Nitro, C₁-C₄Halogenalkyl, C₁-C₆-Alkyl, C₁-C₆-Alkoxy, C₁-C₆-Alkoxycarbonyl oder Formyl, C₁-C₆-Alkylcarbonyl, Benzoyl, C₁-C₆-Alkylsulfonyl, C₁-C₆-Alkoxysulfonyl, Phenylsulfonyl, Phenoxysulfonyl, wobei Benzoyl und Phenylsulfonyl durch Halogen, Methyl, Methoxy oder Ethoxy substituiert sein können, ringsubstituiertes Naphthyl, Phenyl, Naphthyl-C₁-C₆-Alkyl oder Phenyl-C₁-C₄-alkyl; Benzoyl, Formyl, C₁-C₆-Alkylcarbonyl, C₁-C₆-Alkylsulfonyl, C₁-C₆-Alkoxysulfonyl, Phenylsulfonyl oder Phenoxysulfonyl, wobei Benzoyl und Phenylsulfonyl durch Halogen, Methyl, Methoxy oder Ethoxy substituiert sein können; N-C₁-C₆-Alkylcarbamoyl; oder gegebenenfalls ringsubstituiertes N-Naphthylcarbamoyl oder N-Phenylcarbamoyl;
R₁, R₂ und R₃ unabhängig voneinander Wasserstoff; C₁-C₆-Alkyl; C₁-C₆-Alkoxy; C₁-C₆-Alkylthio;C₁-C₆-Alkylsulfonyl; Sulfo; Hydroxy; Nitro; Halogen; Amino; Mono-C₁-C₆-Alkylamino; Di-C₁-C₆-Alkylamino; Naphthylamino oder Phenylamino; Naphthyl-C₁-C₆-alkylamino; Phenyl-C₁-C₆-alkylamino; Dinaphthyl-oder Diphenylamino; (Naphthyl-C₁-C₆-alkyl)-alkylamino oder (Phenyl-C₁-C₆-alkyl)-alkylamino;(Naphthyl-C₁-C₆-alkyl)-C₁-C₆-alkylamino oder (Phenyl-C₁-C₆-alkyl)-C₁-C₆-alkylamino; Di-(naphthyl-C₁-C₆-alkyl)- oder Di-(phenyl-C₁-C₆-alkyl)-amino; oder einen über ein Ringstickstoffatom gebundenen Pyrrolidino-, Piperidino-, Pipecolino-, Morpholino-, Thiomorpholino-, Piperazino-, N-Methylpiperazino- oder N-Phenylpiperazinorest;
R₂ und R₃ gemeinsam eine unsubstituierte oder durch C₁-C₆-Alkyl substituierte vicinal gebundene Methylendioxy- oder 1,2-Ethylendioxygruppe;
R₄, R₅ und R₆ unabhängig voneinander Wasserstoff; Halogen; Cyano; Nitro; C₁-C₆-Alkyl; C₁-C₆-Alkylthio; C₁-C₆-Alkylcarbonyl; C₁-C₆-Alkoxycarbonyl; Amino; Mono-C₁-C₆-Alkylamino; Di-C₁-C₆-Alkylamino; Naphthyl- oder Phenylamino; Naphthyl-oder Phenyl-C₁-C₆-alkylamino; Dinaphthyl- oder Diphenylamino; Naphthyl- oder Phenyl-C₁-C₆-alkylamino; (Naphthyl-C₁-C₆-alkyl)- oder (Phenyl-C₁-C₆-alkyl)-C₁-C₆-alkylamino; Di-(naphthyl-C₁-C₆-alkyl)- oder Di-(phenyl-C₁-C₆-alkyl)-amino; C₅-C₁₀-Cycloalkylamino; DiC₅-C₁₀-cycloalkylamino; C₁-C₆-Alkyl-C₅-C₁₀-cycloalkylamino; C₁-C₆-Alkoxy; C₁-C₆-Alkylthio; C₁-C₆-Alkylsulfonyl; oder einen über ein Ringstickstoffatom gebundenen Pyrrolidino-, Piperidino-, Pipecolino-, Morpholino-, Thiomorpholino-, Piperazino-, N-Methylpiperazino- oder N-Phenylpiperazinorest;
X₁ und X₂, unabhängig voneinander, Wasserstoff; unsubstituiertes oder durch Halogen, Hydroxy, Cyano, Tetrahydrofuryl oder C₁-C₆-Alkoxy substituiertes Alkyl mit höchstens 12 Kohlenstoffatomen; Formyl, C₁-C₆-Alkylcarbonyl, Benzoyl, C₁-C₆-Alkylsulfonyl, C₁-C₆-Alkoxysulfonyl, Phenylsulfonyl, Phenoxysulfonyl, wobei Benzoyl und Phenylsulfonyl durch Halogen, Methyl, Methoxy oder Ethoxy substituiert sein können; Cycloalkyl mit 5 bis 10 Kohlenstoffatomen; oder unsubstituiertes oder durch Halogen, Cyano, Nitro, Trifluormethyl, C₁-C₆-Alkyl, C₁-C₆-Alkoxy, C₁-C₆-Alkoxycarbonyl, -NX'X" oder 4-X'X"N-phenylamino ringsubstituiertes Naphthyl-C₁-C₆-alkyl, Phenyl-C₁-C₆-alkyl, Naphthyl oder Phenyl, worin X' und X", unabhängig voneinander, Wasserstoff, C₁-C₆-Alkyl, Cyclohexyl, Benzyl oder Phenyl darstellen; oder
X₁ und X₂ zusammen mit dem gemeinsamen Stickstoffatom einen Pyrrolidino-, Piperidino-, Pipecolino-, Morpholino-, Thiomorpholino-, Piperazino-, N-Methylpiperazino- oder N-Phenylpiperazinorest
bedeuten.

2. Verbindung der Formel I, nach Anspruch 1 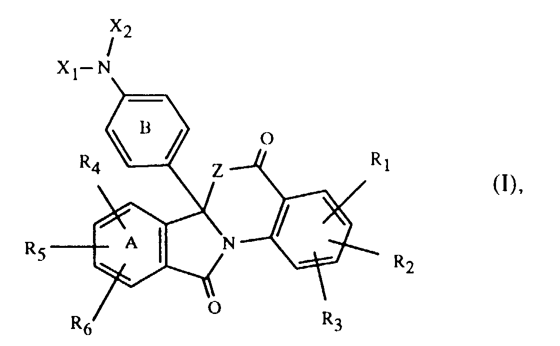 worin
der Ring B einen gegebenenfalls durch Halogen, C₁-C₆-Alkyl, C₁-C₆-Alkoxy, oder Benzyloxy substituierten Benzolkern;
Z 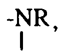 oder O;
R Wasserstoff; unsubstituiertes oder durch Halogen oder C₁-C₆-Alkoxy substituiertes C₁-C₆-Alkyl; Cycloalkyl mit 5 bis 10 Kohlenstoffatomen; unsubstituiertes oder durch Halogen, Cyano, Nitro, C₁-C₄Halogenalkyl, C₁-C₆-Alkyl oder C₁-C₆-Alkoxy ringsubstituiertes Phenyl oder Benzyl; oder Benzoyl, Formyl, C₁-C₆-Alkylcarbonyl, C₁-C₆-Alkylsulfonyl, C₁-C₆-Alkoxysulfonyl, Phenylsulfonyl oder Phenoxysulfonyl, wobei Benzoyl und Phenylsulfonyl durch Halogen, Methyl, Methoxy oder Ethoxy substituiert sein können;
R₁, R₂ und R₃ unabhängig voneinander Wasserstoff; C₁-C₆-Alkyl; C₁-C₆-Alkoxy; C₁-C₆-Alkylthio;C₁-C₆-Alkylsulfonyl; Sulfo; Hydroxy; Nitro; Halogen; Amino; Mono-C₁-C₆-Alkylamino; Di-C₁-C₆-Alkylamino; Naphthylamino oder Phenylamino; Naphthyl-C₁-C₆-alkylamino; Phenyl-C₁-C₆-alkylamino; Dinaphthyl-oder Diphenylamino; (Naphthyl-C₁-C₆-alkyl)-alkylamino oder (Phenyl-C₁-C₆-alkyl)-alkylamino;(Naphthyl-C₁-C₆-alkyl)-C₁-C₆-alkylamino oder (Phenyl-C₁-C₆-alkyl)-C₁-C₆-alkylamino; Di-(naphthyl-C₁-C₆-alkyl)-amino oder Diphenyl-C₁-C₆-alkylamino; oder einen über ein Ringstickstoffatom gebundenen Pyrrolidino-, Piperidino-, Pipecolino-, Morpholino-, Thiomorpholino-, Piperazino-, N-Methylpiperazino- oder N-Phenylpiperazinorest;
R₂ und R₃ gemeinsam eine unsubstituierte oder durch C₁-C₆-Alkyl substituierte vicinal gebundene Methylendioxy- oder 1,2-Ethylendioxygruppe;
R₄, R₅ und R₆ unabhängig voneinander Wasserstoff; Halogen; Cyano; Nitro; C₁-C₆-Alkyl; C₁-C₆-Alkylthio; C₁-C₆-Alkylcarbonyl; C₁-C₆-Alkoxycarbonyl; Amino; Mono-C₁-C₆-Alkylamino; Di-C₁-C₆-Alkylamino; Naphthyl- oder Phenylamino; Naphthyl-oder Phenyl-C₁-C₆-alkylamino; Dinaphthyl- oder Diphenylamino; (Naphthyl-C₁-C₆-alkyl)-amino oder (Phenyl-C₁-C₆-alkyl)amino; (Naphthyl-C₁-C₆-alkyl)- oder (Phenyl-C₁-C₆-alkyl)-C₁-C₆-alkylamino; Di-(naphthyl-C₁-C₆-alkyl)- oder Di-(phenyl-C₁-C₆-alkyl)-amino; C₅-C₁₀-Cycloalkylamino; DiC₅-C₁₀-cycloalkylamino; C₁-C₆-Alkyl-C₅-C₁₀-cycloalkylamino; C₁-C₆-Alkoxy; C₁-C₆-Alkylthio; C₁-C₆-Alkylsulfonyl; oder einen über ein Ringstickstoffatom gebundenen Pyrrolidino-, Piperidino-, Pipecolino-, Morpholino-, Thiomorpholino-, Piperazino-, N-Methylpiperazino- oder N-Phenylpiperazinorest;
X₁ und X₂, unabhängig voneinander, Wasserstoff; unsubstituiertes oder durch Halogen, Hydroxy, Cyano, Tetrahydrofuryl oder C₁-C₆-Alkoxy substituiertes Alkyl mit höchstens 12 Kohlenstoffatomen; Formyl, C₁-C₆-Alkylcarbonyl, Benzoyl, C₁-C₆-Alkylsulfonyl, C₁-C₆-Alkoxysulfonyl, Phenylsulfonyl, Phenoxysulfonyl, wobei Benzoyl und Phenylsulfonyl durch Halogen, Methyl, Methoxy oder Ethoxy substituiert sein können; Cycloalkyl mit 5 bis 10 Kohlenstoffatomen; oder unsubstituiertes oder durch Halogen, Cyano, Nitro, Trifluormethyl oder C₁-C₆-Alkyl, ringsubstituiertes Naphthyl-C₁-C₆-alkyl, Phenyl-C₁-C₆-alkyl, Naphthyl oder Phenyl; oder
X₁ und X₂ zusammen mit dem gemeinsamen Stickstoffatom einen Pyrrolidino-, Piperidino-, Pipecolino-, Morpholino-, Thiomorpholino-, Piperazino-, N-Methylpiperazino- oder N-Phenylpiperazinorest
bedeuten.

3. Verbindung der Formel I, nach Anspruch 1 oder 2 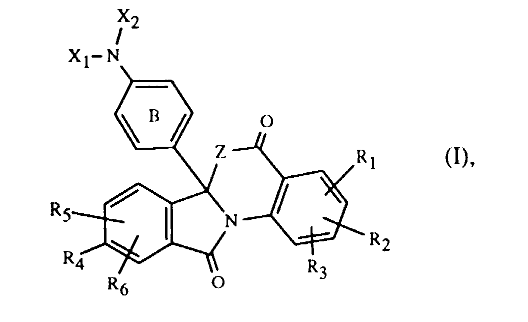 worin
der Ring B einen unsubstituierten oder gegebenenfalls durch Halogen oder C₁-C₄Alkyl, substituierten Benzolkern;
Z 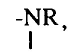 oder O;
R Wasserstoff;
R₁ Wasserstoff; C₁-C₄Alkyl; C₁-C₄Alkoxy; Sulfo; Nitro; Halogen; Mono-C₁-C₄Alkyl-amino; Di-C₁-C₄Alkylamino; Phenylamino; Phenyl-C₁-C₄Alkylamino; Diphenylamino; Benzylamino; Benzyl-C₁-C4Alkylamino; Dibenzylamino;
R₂ und R₃ unabhängig voneinander Wasserstoff; C₁-C₄Alkyl; oder Halogen;
R₂ und R₃ gemeinsam eine vicinal gebundene Methylendioxy- oder 1,2-Ethylendioxygruppe;
R₄ Wasserstoff; Amino; Mono-C₁-C₄Alkylamino; Di-C₁-C₄Alkylamino; Phenylamino; Phenyl-C₁-C₄Alkylamino; Diphenylamino; Benzylamino; Benzyl-C₁-C₄Alkylamino; Dibenzylamino; C₅-C₁₀-Cycloalkylamino; DiC₅-C₁₀-cycloalkylamino; oder C₁-C₄Alkyl-C₅-C₁₀-cycloalkylamino;
R₅ und R₆ unabhängig voneinander Wasserstoff; Halogen; Cyano; Nitro; C₁-C₄Alkyl; C₁-C₄Alkylthio; oder C₁-C₄Alkoxy;
X₁ und X₂, unabhängig voneinander, Wasserstoff; unsubstituiertes oder durch C₁-C₄Alkoxy substituiertes Alkyl mit höchstens 12 Kohlenstoffatomen; Cyclohexyl; der unsubstituiertes oder durch Halogen, C₁-C₄Alkyl, C₁-C₄Alkoxy, ringsubstituiertes Benzyl oder Phenyl; oder
X₁ und X₂ zusammen mit dem gemeinsamen Stickstoffatom einen Pyrrolidino-, Piperidino-, Pipecolino-, Morpholino-, Thiomorpholino-, Piperazino-, N-Methylpiperazino- oder N-Phenylpiperazinorest
bedeuten.

4. Verbindungen der Formel I, nach einem der Ansprüche 1 bis 3, worin
Z O bedeutet.

5. Verbindung der Formel I, nach einem der Ansprüche 1 bis 4, worin
R₁ wie zuvor definiert ist,
R₂ Wasserstoff, Halogen oder C₁-C₄Alkyl und
R₃ Halogen oder Wasserstoff bedeutet.

6. Verbindung der Formel I, nach einem der Ansprüche 1 bis 4, worin
R₁ Mono-C₁-C₄Alkylamino; Di-C₁-C₄Alkylamino;
R₂ Wasserstoff oder C₁-C₄Alkyl und
R₃ Wasserstoff bedeutet

7. Verbindung der Formel I, nach einem der Ansprüche 1 bis 6, worin
R₄ C₁-C₄Alkoxy; Mono-C₁-C₄Alkylamino; Di-C₁-C₄Alkylamino; Phenylamino; Phenyl-C₁-C₄Alkylamino; Diphenylamino; Benzylamino; Benzyl-C₁-C₄Alkylamino; Dibenzylamino; C₅-C₁₀-Cycloalkylamino; DiC₅-C₁₀-cycloalkylamino; oder C₁-C₄Alkyl-C₅-C₁₀-cycloalkylamino bedeutet.

8. Verbindung der Formel I, nach einem der Ansprüche 1 bis 6, worin
R₄ Mono-C₁-C₄Alkylamino, Di-C₁-C₄Alkylamino, Phenylamino, Phenyl-C₁-C₄Alkylamino, Diphenylamino, Benzylamino, Benzyl-C₁-C₄Alkylamino oder Dibenzylamino bedeutet.

9. Verbindung der Formel I, nach einem der Ansprüche 1 bis 8,worin
X₁ und X₂, unabhängig voneinander, Wasserstoff, unsubstituiertes Alkyl mit höchstens 6 Kohlenstoffatomen, Benzyl oder Phenyl bedeutet.

10. Verbindung der Formel I, nach einem der Ansprüche 1 bis 8, worin
X₁ und X₂, unabhängig voneinander, unsubstituiertes Alkyl mit höchstens 6 Kohlenstoffatomen, Benzyl oder Phenyl;
bedeutet.

11. Verfahren zur Herstellung von Lactamverbindungen der Formel (I) gemäss einem oder mehreren der Ansprüche 1 bis 10, dadurch gekennzeichnet, dass man eine Ketosäure der Formel 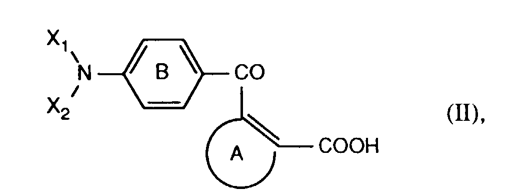 worin X₁, X₂, A und B wie zuvor definiert sind, mit einer bifunktionellen Aminoverbindung der Formel 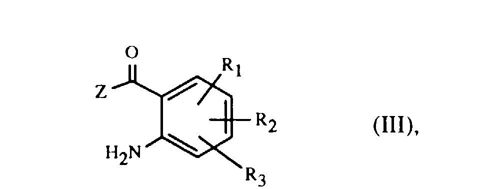 worin R₁, R₂, R₃ und Z wie zuvor definiert sind, umsetzt.

12. Druckempfindliches Aufzeichnungsmaterial enthaltend mindestens einen Farbbildner der Formel (I) gemäss einem oder mehreren der Ansprüche 1 bis 10.

13. Wärmeempfindliches Aufzeichnungsmaterial enthaltend mindestens einen Farbbildner der Formel (I) gemäss einem oder mehreren der Ansprüche 1 bis 10.

14. Mikrokapseln für druckempfindliche Aufzeichnungsmaterialien, enthaltend mindestens einen Farbbildner der Formel (I) gemäss einem oder mehreren der Ansprüche 1 bis 10.

15. Verfahren zur Herstellung mikroverkapselter Farbbildner, dadurch gekennzeichnet, dass man mindestens eine Verbindung der Formel (I) gemäss einem oder mehreren der Ansprüche 1 bis 10 mikroverkapselt.

16. Verfahren zur Herstellung eines druckempfindlichen Aufzeichungsmaterials, dadurch gekennzeichnet, dass man auf einen ersten Träger Mikrokapseln enthaltend mindestens eine Verbindung der Formel (I) nach einem der Ansprüche 1 bis 10 aufträgt und auf den ersten Träger oder auf einen zweiten Träger, der in unmittelbaren Kontakt zu dem ersten Träger angeordnet ist, einen Entwickler aufträgt.

17. Verfahren zur Herstellung eines wärmeempfindlichen Aufzeichungsmaterials, dadurch gekennzeichnet, dass man auf einen Träger eine Mischung von Farbbildnern enthaltend mindestens eine Verbindung der Formel (I) nach einem der Ansprüche 1 bis 10 und einen Entwickler aufträgt.

## Claims

1. A lactam compound of general formula I 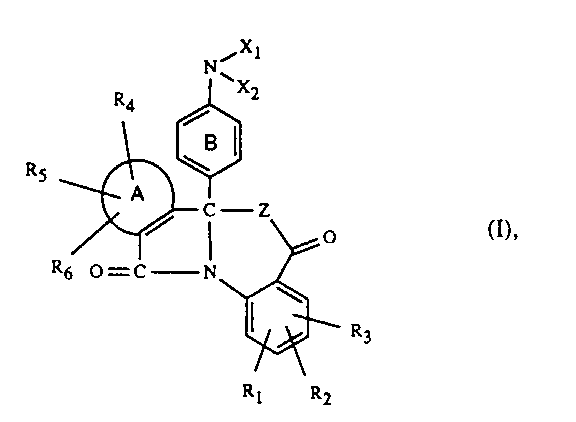 wherein
the ring A is an aromatic or heteroaromatic radical which contains 6 ring atoms and may have a fused-on benzene ring;
the ring B is a benzene nucleus which may be substituted by halogen, C₁-C₆alkyl, C₁-C₆alkoxy, benzyloxy, C₁-C₆alkylcarbonylamino, mono- or di- C₁-C₆alkylamino;
Z is 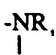 or O;
R is hydrogen; C₁-C₆alkyl which is unsubstituted or substituted by halogen, hydroxy, cyano, benzoyl, formyl, C₁-C₆alkoxycarbonyl, C₁-C₆alkylsulfonyl, C₁-C₆alkoxysulfonyl, phenylsulfonyl or phenoxysulfonyl; it being possible for benzoyl and phenylsulfonyl to be substituted by halogen, methyl, methoxy or ethoxy, di-C₁-C₆alkylamino or C₁-C₆alkoxy; cycloalkyl of 5 to 10 carbon atoms; naphthyl, phenyl, naphthyl-C₁-C₆alkyl or phenyl C₁-C₆alkyl which are each unsubstituted or ring-substituted by halogen, cyano, nitro, C₁-C₄haloalkyl, C₁-C₆alkyl, C₁-C₆alkoxy, C₁-C₆alkoxycarbonyl or formyl, C₁-C₆alkoxycarbonyl, C₁-C₆alkylsulfonyl, C₁-C₆alkoxysulfonyl, phenylsulfonyl - or phenoxysulfonyl; it being possible for benzoyl and phenylsulfonyl to be substituted by halogen, methyl, methoxy or ethoxy; benzoyl, formyl, C₁-C₆alkylcarbonyl, C₁-C₆alkylsulfonyl, C₁-C₆alkoxysulfonyl, phenylsulfonyl or phenoxysulfonyl, it being possible for benzoyl and phenylsulfonyl to be substituted by halogen, methyl, ethoxy or ethoxy; N-C₁-C₆-alkylcarbamoyl; or unsubstituted or ring-substituted N-naphthylcarbamoyl or N-phenylcarbamoyl;
R₁, R₂ and R₃ are each independently of one another hydrogen; C₁-C₆alkyl, C₁-C₆alkoxy; C₁-C₆alkylthio; C₁-C₆alkylsulfonyl; sulfo; hydroxy; nitro; halogen, amino; mono-C₁-C₆alkylamino; di-C₁-C₆alkylamino; naphthylamino or phenylamino; naphthyl-C₁-C₆alkylamino; phenyl-C₁-C₆alkylamino; dinaphthylamino or diphenylamino; (naphthyl-C₁-C₆alkyl)alkylamino or (phenyl-C₁-C₆alkyl)alkylamino; (naphthyl-C₁-C₆alkyl)-C₁-C₆alkylamino or (phenyl-C₁-C₆alkyl)-C₁-C₆alkylamino; di(naphthyl-C₁-C₆alkyl)amino or di(phenyl-C₁-C₆alkyl)amino; or a pyrrolidino, piperidino, pipecolino, morpholino, thiomorpholino, piperazino, N-methylpiperazino or N-phenylpiperazino radical which is attached via a ring nitrogen atom;
R₂ and R₃ together are an unsubstituted or C₁-C₆alkyl-substituted, vicinally attached methylenedioxy or 1,2-ethylenedioxy group;
R₄, R₅ and R₆ are each independently of one another hydrogen; halogen; cyano; nitro; C₁-C₆alkyl; C₁-C₆alkylthio; C₁-C₆alkylcarbonyl; C₁-C₆alkoxycarbonyl; amino; mono-C₁-C₆alkylamino; di-C₁-C₆alkylamino; naphthylamino or phenylamino; naphthyl- or phenyl-C₁-C₆alkylamino; dinaphthylamino or diphenylamino; naphthyl- or phenyl-C₁-C₆alkylamino; (naphthyl-C₁-C₆alkyl)- or (phenyl-C₁-C₆-alkyl)-C₁-C₆alkylamino; di(naphthyl-C₁-C₆alkyl)-or di(phenyl-C₁-C₆alkyl)amino; C₅-C₁₀cycloalkylamino; di-C₅-C₁₀cycloalkylamino; C₁-C₆alkyl-C₅-C₁₀cycloalkylamino; C₁-C₆alkoxy; C₁-C₆alkylsulfonyl; or a pyrrolidino, piperidino, pipecolino, morpholino, thiomorpholino, piperazino, N-methylpiperazino or N-phenylpiperazino radical which is attached via a ring nitrogen atom;
X₁ and X₂ are each independently of one another hydrogen; alkyl of not more than 12 carbon atoms which is unsubstituted or substituted by halogen, hydroxy, cyano, tetrahydrofuryl or C₁-C₆alkoxy; formyl, C₁-C₆alkylcarbonyl, benzoyl, C₁-C₆alkyl-sulfonyl, C₁-C₆alkoxysulfonyl, phenylsulfonyl, phenoxysulfonyl, it being possible for benzoyl and phenylsulfonyl to be substituted by halogen, methyl, methoxy or ethoxy; cycloalkyl of 5 to 10 carbon atoms; or naphthyl-C₁-C₆alkyl, phenyl-C₁-C₆alkyl, naphthyl or phenyl each of which is unsubstituted or ring-substituted by halogen, cyano, nitro, trifluoromethyl, C₁-C₆alkyl, C₁-C₆alkoxy, C₁-C₆alkoxycarbonyl, -NX'X" or 4-X'X"N-phenylamino, in which X' and X" are each independently of the other hydrogen, C₁-C₆alkyl, cyclohexyl, benzyl or phenyl; or
X₁ and X₂ together with the conjoint nitrogen atom are a pyrrolidino, piperidino, pipecolino, morpholino, thiomorpholino, piperazino, N-methylpiperazino or N-phenylpiperazino radical.

2. A compound of formula I according to claim 1 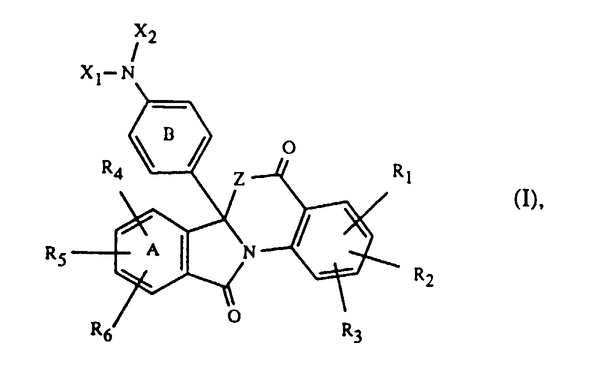 wherein
the ring B is a benzene nucleus which may be substituted by halogen, C₁-C₆alkyl, C₁-C₆alkoxy or benzyloxy;
Z is 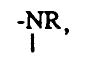 or O;
R is hydrogen; C₁-C₆alkyl which is unsubstituted or substituted by halogen or C₁-C₆alkoxy; cycloalkyl of 5 to 10 carbon atoms; phenyl or benzyl each of which is unsubstituted or ring-substituted by halogen, cyano, nitro, C₁-C₄haloalkyl, C₁-C₆alkyl or C₁-C₆alkoxy; or benzoyl, formyl, C₁-C₆alkylcarbonyl, C₁-C₆alkoxysulfonyl, C₁-C₆alkoxysulfonyl, phenylsulfonyl or phenoxysulfonyl, it being possible for benzoyl and phenylsulfonyl to be substituted by halogen, methyl, methoxy or ethoxy;
R₁, R₂ and R₃ are each independently of one another hydrogen; C₁-C₆alkyl, C₁-C₆alkoxy; C₁-C₆alkylthio; C₁-C₆alkylsulfonyl; sulfo; hydroxy; nitro; halogen, amino; mono-C₁-C₆alkylamino; di-C₁-C₆alkylamino; naphthylamino or phenylamino; naphthyl- or phenyl-C₁-C₆alkylamino; dinaphthylamino or diphenylamino; (naphthyl-C₁-C₆alkyl)alkylamino or (phenyl-C₁-C₆alkyl)alkylamino; (naphthyl-C₁-C₆alkyl)- or (phenyl-C₁-C₆alkyl)-C₁-C₆alkylamino; di(naphthyl-C₁-C₆alkyl)- or di(phenyl-C₁-C₆alkyl)amino; or pyrrolidino, piperidino, pipecolino, morpholino, thiomorpholino, piperazino, N-methylpiperazino or N-phenylpiperazino radical which is attached via a ring nitrogen atom;
R₂ and R₃ together are an unsubstituted or C₁-C₆alkyl-substituted, vicinally attached methylenedioxy or 1,2-ethylenedioxy group;
R₄, R₅ and R₆ are each independently of one another hydrogen; halogen; cyano; nitro; C₁-C₆alkyl; C₁-C₆alkylthio; C₁-C₆alkylcarbonyl; C₁-C₆alkoxycarbonyl; amino; mono-C₁-C₆alkylamino; di-C₁-C₆alkylamino; naphthylamino or phenylamino; naphthyl- or phenyl-C₁-C₆alkylamino; dinaphthylamino or diphenylamino; naphthyl- or phenyl-C₁-C₆alkylamino; (naphthyl-C₁-C₆alkyl)- or (phenyl-C₁-C₆-alkyl)-C₁-C₆alkylamino; di(naphthyl-C₁-C₆alkyl)-or di(phenyl-C₁-C₆alkyl)amino; C₅-C₁₀cycloalkylamino; di-C₅-C₁₀cycloalkylamino; C₁-C₆alkyl-C₅-C₁₀cycloalkylamino; C₁-C₆alkoxy; C₁-C₆alkylsulfonyl; or a pyrrolidino, piperidino, pipecolino, morpholino, thiomorpholino, piperazino, N-methylpiperazino or N-phenylpiperazino radical which is attached via a ring nitrogen atom;
X₁ and X₂ are each independently of one another hydrogen; alkyl of not more than 12 carbon atoms which is unsubstituted or substituted by halogen, hydroxy, cyano, tetrahydrofuryl or C₁-C₆alkoxy; formyl, C₁-C₆alkylcarbonyl, benzoyl, C₁-C₆alkylsulfonyl, C₁-C₆alkoxysulfonyl, phenylsulfonyl or phenoxysulfonyl, it being possible for benzoyl and phenylsulfonyl to be substituted by halogen, methyl, methoxy or ethoxy; cycloalkyl of 5 to 10 carbon atoms; or naphthyl-C₁-C₆alkyl, phenyl-C₁-C₆alkyl, naphthyl or phenyl each of which is unsubstituted or ring-substituted by halogen, cyano, nitro, trifluoromethyl or C₁-C₆alkyl;
X₁ and X₂ together with the conjoint nitrogen atom are a pyrrolidino, piperidino, pipecolino, morpholino, thiomorpholino, piperazino, N-methylpiperazino or N-phenylpiperazino radical.

3. A compound of formula I according to claim 1 or 2 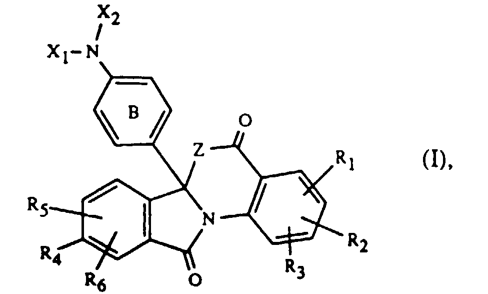 wherein
the ring B is a benzene nucleus which is unsubstituted or may be substituted by halogen or C₁-C₄alkyl;
Z is 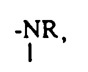 or O;
R is hydrogen;
R₁ is hydrogen; C₁-C₄alkyl; C₁-C₄alkoxy; sulfo; nitro; halogen; mono-C₁-C₄alkylamino; di-C₁-C₄alkylamino; phenylamino; phenyl-C₁-C₄alkylamino; diphenylamino; benzylamino; benzyl-C₁-C₄alkylamino; dibenzylamino;
R₂ and R₃ are each independently of one another hydrogen; C₁-C₄alkyl; or halogen;
R₂ and R₃ together are a vicinally attached methylenedioxy or 1,2-ethylenedioxy group;
R₄ is hydrogen; amino; mono-C₁-C₄alkylamino; di-C₁-C₄alkylamino; phenylamino; phenyl-C₁-C₄alkylamino; diphenylamino; benzylamino; benzyl-C₁-C₄alkylamino; dibenzylamino; C₅-C₁₀cycloalkylamino; di-C₅-C₁₀cycloalkylamio; or C₁-C₄alkyl-C₅-C₁₀cycloalkylamino;
R₅ and R₆ are each independently of one another hydrogen; halogen; cyano; nitro; C₁-C₄alkyl; C₁-C₄alkylthio; or C₁-C₄alkoxy;
X₁ and X₂ are each independently of one another hydrogen; unsubstituted or C₁-C4alkoxy-substituted alkyl of not more than 12 carbon atoms; cyclohexyl; benzyl or phenyl each of which are unsubstituted or ring-substituted by halogen, C₁-C₄alkyl or C₁-C₄alkoxy; or
X₁ and X₂ together with the conjoint nitrogen atom are a pyrrolidino, piperidino, pipecolino, morpholino, thiomorpholino, piperazino, N-methylpiperazino or N-phenylpiperazino radical.

4. A compound of formula I according to any one of claims 1 to 3, wherein
Z is 0.

5. A compound of formula I according to any one of claims 1 to 4, wherein
R₁ is as previously defined,
R₂ is hydrogen, halogen or C₁-C₄alkyl, and
R₃ is halogen or hydrogen.

6. A compound of formula I according to any one of claims 1 to 4, wherein
R₁ is mono-C₁-C₄alkylamino or di-C₁-C₄alkylamino;
R₂ is hydrogen or C₁-C₄alkyl, and
R₃ is hydrogen.

7. A compound of formula I according to any one of claims 1 to 6, wherein
R₄ is C₁-C₄alkoxy; mono-C₁-C₄alkylamino; di-C₁-C₄alkylamino; phenylamino; phenyl-C₁-C₄alkylamino; diphenylamino; benzylamino; benzyl-C₁-C₄alkylamino; dibenzylamino; C₅-C₁₀cycloalkylamino; di-C₅-C₁₀cycloalkylamino; or C₁-C₄alkyl-C₅-C₁₀cycloalkylamino.

8. A compound of formula I according to any one of claims 1 to 6, wherein
R₄ is mono-C₁-C₄alkylamino, di-C₁-C₄alkylamino, phenylamino, phenyl-C₁-C₄alkylamino, diphenylamino, benzylamino, benzyl-C₁-C₄alkylamino or dibenzylamino.

9. A compound of formula I according to any one of claims 1 to 8, wherein
X₁ and X₂ are each independently of one another hydrogen, unsubstituted alkyl of not more than 6 carbon atoms, benzyl or phenyl.

10. A compound of formula I according to any one of claims 1 to 8, wherein
X₁ and X₂ are each independently of one another unsubstituted alkyl of not more than 6 carbon atoms, benzyl or phenyl.

11. A process for the preparation of a lactam compound of formula (I) according to one or more of claims 1 to 10, which comprises reacting a keto acid of formula 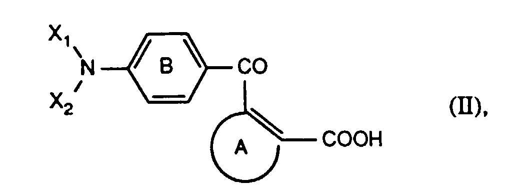 in which X₁, X₂, A and B are as previously defined with a bifunctional amino compound of formula 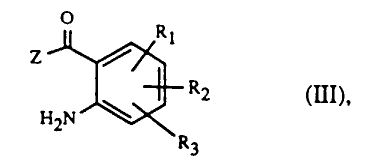 in which R₁, R₂, R₃ and Z are as previously defined.

12. A pressure-sensitive recording material comprising at least one colour former of formula (I) according to one or more of claims 1 to 10.

13. A heat-sensitive recording material comprising at least one colour former of formula (I) according to one or more of claims 1 to 10.

14. Microcapsules for a pressure-sensitive recording material comprising at least one colour former of formula (I) according to one or more of claims 1 to 10.

15. A process for the preparation of a microencapsulated colour former, which comprises microencapsulating at least one compound of formula (I) according to one or more of claims 1 to 10.

16. A process for the preparation of a pressure-sensitive recording material, which comprises applying to a first substrate microcapsules comprising at least one compound of formula (I) according to any one of claims 1 to 10, and applying a developer to the first substrate or to a second substrate which is in direct contact with said first substrate.

17. A process for the preparation of a heat-sensitive recording material, which comprises applying to a substrate a mixture of colour formers comprising at least one compound of formula (I) according to one or more of claims 1 to 10 and a developer.

## Revendications

1. Composé du type lactame de formule générale I 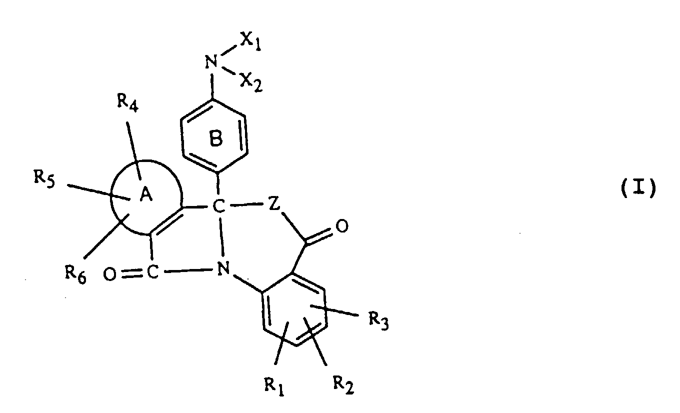 dans laquelle
le cycle A représente un reste aromatique ou hétéroaromatique avec 6 atomes de cycle, lequel peut présenter un cycle benzénique condensé ;
le cycle B représente un noyau benzénique éventuellement substitué par des atomes d'halogènes, des substituants alkyle en C₁-C₆, alkoxy en C₁-C₆, benzyloxy, (alkyl en C₁-C₆)-carbonylamino, mono-ou di-(alkyl en C₁-C₆)-amino ;
Z représente 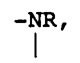 ou O ;
R représente un atome d'hydrogène ; un groupe alkyle en C₁-C₆ non substitué ou substitué par des atomes d'halogènes, des substituants hydroxy, cyano, benzoyle, formyle, (alkyl en C₁-C₆)carbonyle, (alkyl en C₁-C₆)-sulfonyle, (alkoxy en C₁-C₆)-sulfonyle, phénylsulfonyle, phénoxysulfonyle, les restes benzoyle et phénylsulfonyle pouvant être substitués par des atomes d'halogènes, des substituants méthyle, méthoxy ou éthoxy, di(alkyl en C₁-C₆)amino ou alkoxy en C₁-C₆ ; cycloalkyle comportant 5 à 10 atomes de carbone ; naphtyle, phényle, naphtylalkyle en C₁-C₆, ou phénylalkyle en C₁-C₄ non substitué ou substitué sur le cycle par des atomes d'halogènes, des substituants cyano, nitro, haloalkyle en C₁-C₄, alkyle en C₁-C₆, alkoxy en C₁-C₆, (alkoxy en C₁-C₆)carbonyle ou formyle, (alkyl en C₁-C₆)-carbonyle, benzoyle, (alkyl en C₁-C₆)sulfonyle, (alkoxy en C₁-C₆)-sulfonyle, phénylsulfonyle, phénoxysulfonyle, les restes benzoyle et phénylsulfonyle pouvant être substitués par des substituants halogène, méthyle, méthoxy ou éthoxy ; benzoyle, formyle, (alkyl en C₁-C₆)-carbonyle, (alkyl en C₁-C₆)sulfonyle, (alkoxy en C₁-C₆)sulfonyle, phénylsulfonyle ou phénoxysulfonyle, les restes benzoyle et phénylsulfonyle pouvant être substitués par des substituants halogène, méthyle, méthoxy ou éthoxy ; N-(alkyl en C₁-C₆)carbamoyle ; ou N-naphtylcarbamoyle ou N-phénylcarbamoyle éventuellement substitué sur le cycle ;
R₁, R₂ et R₃ représentent indépendamment les uns des autres des atomes d'hydrogène ; des groupes alkyle en C₁-C₆ ; alkoxy en C₁-C₆ ; (alkyl en C₁-C₆)thio ; (alkyl en C₁-C₆)sulfonyle ; sulfo ; hydroxy ; nitro ; halogène ; amino ; mono(alkyl en C₁-C₆)-amino ; di-(alkyl en C₁-C₆)amino ; naphtylamino ou phénylamino ; naphtyl(alkyl en C₁-C₆)amino ; phényl(alkyl en C₁-C₆)amino ; dinaphtyl- ou diphénylamino ; (naphtylalkyl en C₁-C₆)-alkylamino ou (phénylalkyl en C₁-C₆)-alkylamino ; (naphtylalkyl en C₁-C₆)-(alkyl en C₁-C₆)amino ou (phénylalkyl en C₁-C₆)-(alkyl en C₁-C₆)amino ; di-(naphtylalkyl en C₁-C₆)- ou di-(phénylalkyl en C₁-C₆)-amino ; ou un reste pyrrolidino, pipéridino, pipécolino, morpholino, thio-morpholino, pipérazino, N-méthylpipérazino ou N-phénylpipérazino lié par l'intermédiaire d'un atome d'azote du cycle ;
R₂ et R₃ ensemble représentent un groupe méthylènedioxy ou 1,2-éthylènedioxy lié en position adjacente non substitué ou substitué par un groupe alkyle en C₁-C₆ ;
R₄, R₅ et R₆ représentent indépendamment les uns des autres des atomes d'hydrogène ; des atomes d'halogènes ; des substituants cyano ; nitro ; alkyle en C₁-C₆ ; (alkyl en C₁-C₆)thio ; (alkyl en C₁-C₆)-carbonyle ; (alkoxy en C₁-C₆)carbonyle ; amino ; mono-(alkyl en C₁-C₆)amino ; di-(alkyl en C₁-C₆)-amino ; naphtyl- ou phénylamino ; naphtyl-ou phényl(alkyl en C₁-C₆)amino ; dinaphtyl- ou diphénylamino ; naphtyl- ou phényl(alkyl en C₁-C₆)-amino ; (naphtylalkyl en C₁-C₆)- ou (phénylalkyl en C₁-C₆)-(alkyl en C₁-C₆)amino ; di-(naphtylalkyl en C₁-C₆)- ou di-(phénylalkyl en C₁-C₆)-amino ; (cycloalkyl en C₅-C₁₀)-amino ; di(cycloalkyl en C₅-C₁₀)amino ; (alkyl en C₁-C₆)-(cycloalkyl en C₅-C₁₀)amino ; alkoxy en C₁-C₆ ; (alkyl en C₁-C₆)thio ; (alkyl en C₁-C₆)sulfonyle ; ou un reste pyrrolidino, pipéridino, pipécolino, morpholino, thiomorpholino, pipérazino, N-méthylpipérazino ou N-phénylpipérazino lié par l'intermédiaire d'un atome d'azote du cycle ;
X₁ et X₂ représentent indépendamment l'un de l'autre des atomes d'hydrogène ; des groupes alkyle comportant au maximum 12 atomes de carbone non substitué ou substitué par des atomes d'halogènes, des substituants hydroxy, cyano, tétrahydrofuryle ou alkoxy en C₁-C₆ ; formyle, (alkyl en C₁-C₆)-carbonyle, benzoyle, (alkyl en C₁-C₆)sulfonyle, (alkoxy en C₁-C₆)-sulfonyle, phénylsulfonyle, phénoxysulfonyle, les restes benzoyle et phénylsulfonyle pouvant être substitués par des substituants halogène, méthyle, méthoxy ou éthoxy ; cycloalkyle comportant 5 à 10 atomes de carbone ; ou naphtylalkyle en C₁-C₆, phénylalkyle en C₁-C₆, naphtyle ou phényle non substitué ou substitué sur le cycle par des atomes d'halogènes, des substituants cyano, nitro, trifluorométhyle, alkyle en C₁-C₆, alkoxy en C₁-C₆, (alkoxy en C₁-C₆)-carbonyle, -NX'X"- ou 4-X'X"N-phénylamino, où X' et X" représentent indépendamment l'un de l'autre des atomes d'hydrogène, des groupes alkyle en C₁-C₆, cyclohexyle, benzyle ou phényle ; ou
X₁ et X₂ représentent ensemble avec l'atome d'azote commun un reste pyrrolidino, pipéridino, pipécolino, morpholino, thiomorpholino, pipérazino, N-méthylpipérazino ou N-phénylpipérazino.

2. Composé de formule I, selon la revendication 1 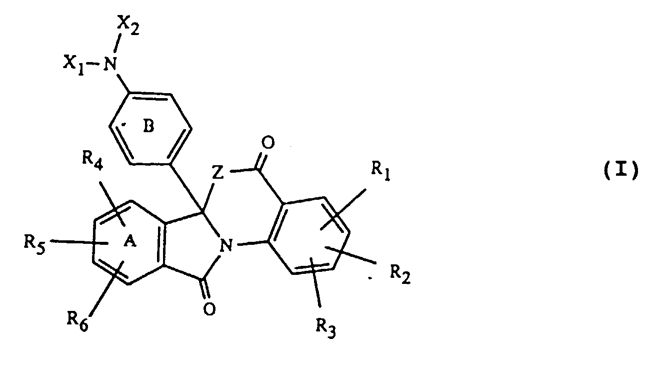 dans laquelle
le cycle B représente un noyau benzénique éventuellement substitué par des atomes d'halogènes, des substituants alkyle en C₁-C₆, alkoxy en C₁-C₆ ou benzyloxy ;
Z représente 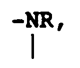 ou O ;
R représente un atome d'hydrogène ; un groupe alkyle en C₁-C₆ non substitué ou substitué par des substituants halogène ou alkoxy en C₁-C₆ ; cycloalkyle comportant 5 à 10 atomes de carbone ; phényle ou benzyle non substitué ou substitué sur le cycle par des atomes d'halogènes, des substituants cyano, nitro, haloalkyle en C₁-C₄ alkyle en C₁-C₆ ou alkoxy en C₁-C₆ ; ou benzoyle, formyle, (alkyl en C₁-C₆)-carbonyle, (alkyl en C₁-C₆)-sulfonyle, (alkoxy en C₁-C₆)sulfonyle, phénylsulfonyle ou phénoxysulfonyle, les restes benzoyle et phénylsulfonyle pouvant être substitués par des atomes d'halogènes, des substituants méthyle, méthoxy ou éthoxy ;
R₁, R₂ et R₃ représentent indépendamment les uns des autres des atomes d'hydrogène ; des substituants alkyle en C₁-C₆ ; alkoxy en C₁-C₆ ; (alkyl en C₁-C₆)thio ; (alkyl en C₁-C₆)sulfonyle ; sulfo ; hydroxy ; nitro ; halogène ; amino ; mono(alkyl en C₁-C₆)-amino ; di-(alkyl en C₁-C₆)amino ; naphtylamino ou phénylamino ; naphtyl(alkyl en C₁-C₆)-amino ; phényl(alkyl en C₁-C₆)amino ; dinaphtyl-ou diphénylamino ; (naphtylalkyl en C₁-C₆)-alkylamino ou (phénylalkyl en C₁-C₆)-alkylamino ; (naphtylalkyl en C₁-C₆)-(alkyl en C₁-C₆)amino ou (phénylalkyl en C₁-C₆)-(alkyl en C₁-C₆)amino ; di-(naphtylalkyl en C₁-C₆)-amino ou di-(phénylalkyl en C₁-C₆)-amino ; ou un reste pyrrolidino, pipéridino, pipécolino, morpholino, thio-morpholino, pipérazino, N-méthylpipérazino ou N-phénylpipérazino lié par l'intermédiaire d'un atome d'azote du cycle ;
R₂ et R₃ ensemble représentent un groupe méthylènedioxy ou 1,2-éthylènedioxy lié en position adjacente, non substitué ou substitué par un groupe alkyle en C₁-C₆ ;
R₄, R₅ et R₆ représentent indépendamment les uns des autres des atomes d'hydrogène ; des atomes d'halogènes ; des substituants cyano ; nitro ; alkyle en C₁-C₆ ; (alkyl en C₁-C₆)thio ; (alkyl en C₁-C₆)-carbonyle ; (alkoxy en C₁-C₆)carbonyle ; amino ; mono-(alkyl en C₁-C₆)amino ; di-(alkyl en C₁-C₆)-amino ; naphtyl- ou phénylamino ; naphtyl-ou phényl(alkyl en C₁-C₆)amino ; dinaphtyl- ou diphénylamino ; naphtyl(alkyl en C₁-C₆)amino ou phényl(alkyl en C₁-C₆)amino ; (naphtylalkyl en C₁-C₆)- ou (phénylalkyl en C₁-C₆)-(alkyl en C₁-C₆)-amino ; di-(naphtylalkyl en C₁-C₆)- ou di-(phénylalkyl en C₁-C₆)amino ; (cycloalkyl en C₅-C₁₀)-amino ; di(cycloalkyl en C₅-C₁₀)amino ; (alkyl en C₁-C₆)-(cycloalkyl en C₅-C₁₀)amino ; alkoxy en C₁-C₆ ; (alkyl en C₁-C₆)thio ; (alkyl en C₁-C₆)sulfonyle ; ou un reste pyrrolidino, pipéridino, pipécolino, morpholino, thio-morpholino, pipérazino, N-méthylpipérazino ou N-phénylpipérazino lié par l'intermédiaire d'un atome d'azote dy cycle ;
X₁ et X₂ représentent indépendamment l'un de l'autre des atomes d'hydrogène ; des groupes alkyle comportant au maximum 12 atomes de carbone non substitué ou substitué par des atomes d'halogènes, des substituants hydroxy, cyano, tétrahydrofuryle ou alkoxy en C₁-C₆ ; formyle, (alkyl en C₁-C₆)-carbonyle, benzoyle, (alkyl en C₁-C₆)sulfonyle, (alkoxy en C₁-C₆)sulfonyle, phénylsulfonyle, phénoxysulfonyle, les restes benzoyle et phénylsulfonyle pouvant être substitués par des substituants halogène, méthyle, méthoxy ou éthoxy ; cycloalkyle comportant 5 à 10 atomes de carbone ; ou naphtylalkyle en C₁-C₆, phénylalkyle en C₁-C₆, naphtyle ou phényle non substitué ou substitué sur le cycle par des atomes d'halogènes, des substituants cyano, nitro, trifluorométhyle ou alkyle en C₁-C₆ ; ou
X₁ et X₂ représentent ensemble avec l'atome d'azote commun un reste pyrrolidino, pipéridino, pipécolino, morpholino, thiomorpholino, pipérazino, N-méthylpipérazino ou N-phénylpipérazino.

3. Composé de formule I, selon la revendication 1 ou 2 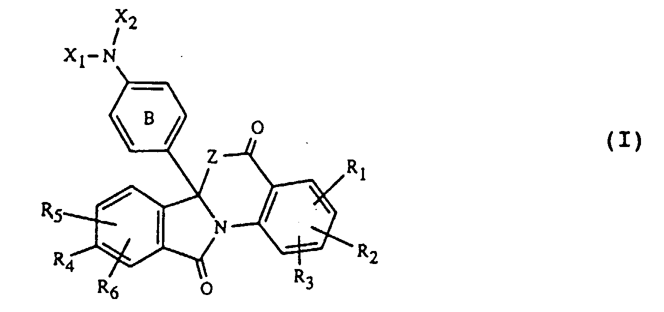 dans laquelle
le cycle B représente un noyau benzénique non substitué ou éventuellement substitué par des atomes d'halogènes ou des groupes alkyle en C₁-C₄ ;
Z représente 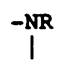 ou O ;
R représente un atome d'hydrogène ;
R₁ représente un atome d'hydrogène ; des groupes alkyle en C₁-C₄ ; alkoxy en C₁-C₄ ; sulfo ; nitro ; halogène ; mono(alkyl en C₁-C₄)amino ; di(alkyl en C₁-C₄) amino ; phényl(alkyl en C₁-C₄)-amino ; diphénylamino ; benzylamino ; benzyl-(alkyl en C₁-C₄)amino ; dibenzylamino ;
R₂ et R₃ représentent indépendamment l'un de l'autre un atome d'hydrogène ; un groupe alkyle en C₁-C₄ ; ou un atome d'halogène ;
R₂ et R₃ représentent ensemble un groupe méthylènedioxy-ou 1,2-éthylènedioxy lié en position adjacente ;
R₄ un atome d'hydrogène ; des groupes amino ; mono(alkyl en C₁-C₄)amino ; di(alkyl en C₁-C₄)-amino ; phénylamino ; phényl(alkyl en C₁-C₄)-amino ; diphénylamino ; benzylamino ; benzyl(alkyl en C₁-C₄)amino ; dibenzylamino ; cyclo(alkyl en C₅-C₁₀)amino ; dicyclo(alkyl en C₅-C₁₀)amino ; ou (alkyl en C₁-C₄)-cyclo(alkyl en C₅-C₁₀)amino ;
R₅ et R₆ représentent indépendamment l'un de l'autre des atomes d'hydrogène ; des atomes d'halogène ; des substituants cyano ; nitro ; alkyle en C₁-C₄ ; (alkyl en C₁-C₄)thio ; ou alkoxy en C₁-C₄ ;
X₁ et X₂ représentent indépendamment l'un de l'autre des atomes d'hydrogène ; un groupe alkyle au maximum comportant 12 atomes de carbone non substitué ou substitué par des groupes alkoxy en C₁-C₄ ; cyclohexyle ; benzyle ou phényle non substitué ou substitué sur le cycle par des atomes d'halogènes, des groupes alkyle en C₁-C₄, alkoxy en C₁-C₄ ; ou
X₁ et X₂ représentent ensemble avec l'atome d'azote commun un reste pyrrolidino, pipéridino, pipécolino, morpholino, thiomorpholino, pipérazino, N-méthylpipérazino ou N-phénylpipérazino.

4. Composés de formule I selon l'une des revendications 1 à 3 où
Z représente O.

5. Composé de formule I selon l'une des revendications 1 à 4, où
R₁ est défini comme précédemment,
R₂ représente un atome d'hydrogène, un atome d'halogène ou un groupe alkyle en C₁-C₄ et
R₃ représente un atome d'halogène ou un atome d'hydrogène.

6. Composés de formule I, selon l'une des revendications 1 à 4 où
R₁ représente les groupes mono(alkyl en C₁-C₄)amino, di(alkyl en C₁-C₄)amino ;
R₂ représente un atome d'hydrogène ou un groupe alkyle en C₁-C₄ et
R₃ représente un atome d'hydrogène.

7. Composés de formule I, selon l'une des revendications 1 à 6 où
R₄ représente les groupes alkoxy en C₁-C₄ ; mono(alkyl en C₁-C₄)amino ; di(alkyl en C₁-C₄)-amino ; phénylamino ; phényl(alkyl en C₁-C₄)-amino ; diphénylamino ; benzylamino ; benzyl(alkyl en C₁-C₄)amino ; dibenzylamino ; cyclo(alkyl en C₅-C₁₀)amino ; dicyclo(alkyl en C₅-C₁₀)amino ; ou (alkyl en C₁-C₄)-cyclo(alkyl en C₅-C₁₀)amino.

8. Composés de formule I, selon l'une des revendications 1 à 6 où
R₄ représente les groupes mono(alkyl en C₁-C₄)amino, di(alkyl en C₁-C₄)amino, phénylamino, phényl(alkyl en C₁-C₄)amino, diphénylamino, benzylamino, benzyl(alkyl en C₁-C₄)amino ou dibenzylamino.

9. Composés de formule I, selon l'une des revendications 1 à 8 où
X₁ et X₂ représentent indépendamment l'un de l'autre des atomes d'hydrogène, des groupes alkyle non substitué comportant au maximum 6 atomes de carbone, benzyle ou phényle.

10. Composés de formule I, selon l'une des revendications 1 à 8 où
X₁ et X₂ représentent indépendamment l'un de l'autre des groupes alkyle non substitué comportant au maximum 6 atomes de carbone, benzyle ou phényle.

11. Procédé de préparation des composés du type lactame de formule (I) selon une ou plusieurs des revendications 1 à 10, caractérisé en ce que l'on fait réagir un cétoacide de formule 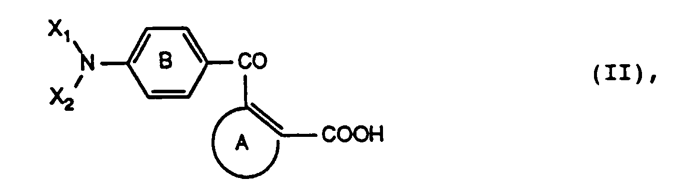 dans laquelle X₁, X₂, A et B sont définis précédemment, avec un composé aminé bifonctionnel de formule 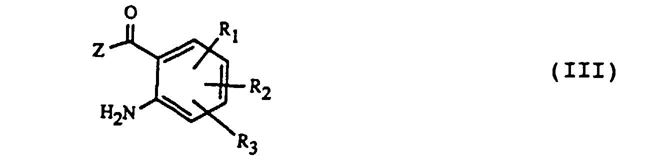 dans laquelle R₁, R₂, R₃ et Z sont définis comme précédemment,

12. Matière d'enregistrement sensible à la pression contenant au moins un coupleur coloré de formule (I) selon une ou plusieurs des revendications 1 à 10.

13. Matière d'enregistrement thermosensible contenant au moins un coupleur coloré de formule (I) selon une ou plusieurs des revendications 1 à 10.

14. Microcapsules pour matières d'enregistrement sensibles à la pression, contenant au moins un coupleur coloré de formule (I) selon une ou plusieurs des revendications 1 à 10.

15. Procédé de préparation de coupleurs colorés microencapsulés, caractérisé en ce que l'on microencapsule au moins un composé de formule (I) selon une ou plusieurs des revendications 1 à 10.

16. Procédé de préparation d'une matière d'enregistrement sensible à la pression, caractérisé en ce que l'on applique, sur un premier support, des microcapsules contenant au moins un composé de formule (I) selon l'une des revendications 1 à 10 et sur le premier support ou un second support, lequel est en contact avec le premier support, on applique un révélateur.

17. Procédé de préparation d'une matière d'enregistrement thermosensible, caractérisé en ce que l'on applique sur un support un mélange de coupleurs colorés contenant au moins un composé de formule (I) selon l'une des revendications 1 à 10 et un révélateur.
